# EUROPEAN PATENT APPLICATION

(11) **EP 3 845 656 A1**
(43) Date of publication of application: **07.07.2021**
(21) Application number: 19855599.7
(22) Date of filing: 28.04.2019
(51) Int. Cl.: C12N 15/867, C12N 5/10

(54) **IMPROVED LENTIVIRAL VECTOR**

(30) Priority: 28.08.2018 CN 201810986752
(71) Applicant: Pharos Vaccine Inc., Gyeonggi-do 13215 (KR); Immunotech Biopharm Co., Ltd., Beijing 100176 (CN)
(72) Inventor: LEE, Hyunsoo, Seongnam-si Gyeonggi-do 13215 (KR); JUNG, Namchul, Seongnam-si Gyeonggi-do 13215 (KR); WANG, Yu, Beijing 100176 (CN)
(74) Representative: Nederlandsch Octrooibureau
(86) International application number: PCT/CN2019/084828
(87) International publication number: WO 2020/042648

(57) **Abstract**

The invention belongs to the field of biomedicine. Specifically, the present invention relates to improved t lentiviral vector, and preparation method and uses thereof. Specifically, the present invention relates to a lentiviral vector especially suitable for preparing a therapeutic T cell.

Sequence Listing

<110>  Pharos Vaccine Inc.
       Immunotech Biopharm Co.,Ltd.

<120>  Improved lentiviral vector

<130>  P6100673PCT-EP

<150>  CN201810986752.8
<151>  2018-08-28

<160>  18

<170>  PatentIn version 3.5

<210>  1
<211>  5320
<212>  DNA
<213>  Artificial Sequence

<220>
<223>  pPVLV1 vector containing CAR-19 coding sequence

<400>  1
gggtctctct ggttagacca gatctgagcc tgggagctct ctggctaact agggaaccca      60

ctgcttaagc ctcaataaag cttgccttga gtgcttcaag tagtgtgtgc ccgtctgttg     120

tgtgactctg gtaactagag atccctcaga cccttttagt cagtgtggaa aatctctagc     180

agtggcgccc gaacagggac ttgaaagcga aagggaaacc agaggagctc tctcgacgca     240

ggactcggct tgctgaagcg cgcacggcaa gaggcgaggg gcggcgactg gtgagtacgc     300

caaaaatttt gactagcgga ggctagaagg agagagatgg gtgcgagagc gtcagtatta     360

agcgggggag aattagatcg cgatgggaaa aaattcggtt aaggccaggg ggaaagaaaa     420

aatataaatt aaaacatata gtatgggcaa gcagggagct agaacgattc gcagttaatc     480

ctggcctgtt agaaacatca gaaggctgta gacaaatact gggacagcta caaccatccc     540

ttcagacagg atcagaagaa cttagatcat tatataatac agtagcaacc ctctattgtg     600

tgcatcaaag gatagagata aaagacacca aggaagcttt agacaagata gaggaagagc     660

aaaacaaaag taagaccacc gcacagcaag cggccggccg ctgatcttca gacctggagg     720

aggagatatg agggacaatt ggagaagtga attatataaa tataaagtag taaaaattga     780

accattagga gtagcaccca ccaaggcaaa gagaagagtg gtgcagagag aaaaaagagc     840

agtgggaata ggagctttgt tccttgggtt cttgggagca gcaggaagca ctatgggcgc     900

agcgtcaatg acgctgacgg tacaggccag acaattattg tctggtatag tgcagcagca     960

gaacaatttg ctgagggcta ttgaggcgca acagcatctg ttgcaactca cagtctgggg    1020

catcaagcag ctccaggcaa gaatcctggc tgtggaaaga tacctaaagg atcaacagct    1080

cctggggatt tggggttgct ctggaaaact catttgcacc actgctgtgc cttggaatgc    1140

tagttggagt aataaatctc tggaacagat ttggaatcac acgacctgga tggagtggga 1200

cagagaaatt aacaattaca caagcttaat acactcctta attgaagaat cgcaaaacca 1260

gcaagaaaag aatgaacaag aattattgga attagataaa tgggcaagtt tgtggaattg 1320

gtttaacata acaaattggc tgtggtatat aaaattattc ataatgatag taggaggctt 1380

ggtaggttta agaatagttt ttgctgtact ttctatagtg aatagagtta ggcagggata 1440

ttcaccatta tcgtttcaga cccacctccc aaccccgagg ggacccgaca ggcccgaagg 1500

aatagaagaa gaaggtggag agagagacag agacagatcc attcgattag tgaacggatc 1560

ggcactgcgt gcgccaattc tgcagacaaa tggcagtatt catccacaat tttaaaagaa 1620

aaggggggat tggggggtac agtgcagggg aaagaatagt agacataata gcaacagaca 1680

tacaaactaa agaattacaa aaacaaatta caaaaattca aaattttcgg gtttattaca 1740

gggacagcag agatccagtt tggttagtac cgggcccgac gtcgcatgct cccggccgcc 1800

atggcggccg cgggaattcg attagatccg gtgcccgtca gtgggcagag cgcacatcgc 1860

ccacagtccc cgagaagttg gggggagggg tcggcaattg aaccggtgcc tagagaaggt 1920

ggcgcggggt aaactgggaa agtgatgtcg tgtactggct ccgccttttt cccgagggtg 1980

ggggagaacc gtatataagt gcagtagtcg ccgtgaacgt tctttttcgc aacgggtttg 2040

ccgccagaac acagctgaag cttcgagggg ctcgcatctc tccttcacgc gcccgccgcc 2100

ctacctgagg ccgccatcca cgccggttga gtcgcgttct gccgcctccc gcctgtggtg 2160

cctcctgaac tgcgtccgcc gtctaggtaa gtttaaagct caggtcgaga ccgggccttt 2220

gtccggcgct cccttggagc ctacctagac tcagccggct ctccacgctt tgcctgaccc 2280

tgcttgctca actctacgtc tttgtttcgt tttctgttct gcgccgttac agatccaagc 2340

tgtgaccggc gcctacgtaa gtgatatcta ctagatttat caaaaagagt gttgacttgt 2400

gagcgctcac aattgatact tagattcatc gagagggaca cgtcgactac taaccttctt 2460

ctctttccta cagctgagat cgccggtggg atcccctagg gttaacatgg ccttaccagt 2520

gaccgccttg ctcctgccgc tggccttgct gctccacgcc gccaggccgg acatccagat 2580

gacacagact acatcctccc tgtctgcctc tctgggagac agagtcacca tcagttgcag 2640

ggcaagtcag gacattagta aatatttaaa ttggtatcag cagaaaccag atggaactgt 2700

taaactcctg atctaccata catcaagatt acactcagga gtcccatcaa ggttcagtgg 2760

cagtgggtct ggaacagatt attctctcac cattagcaac ctggagcaag aagatattgc 2820

cacttacttt tgccaacagg gtaatacgct tccgtacacg ttcggagggg ggaccaagct 2880

ggagatcaca ggtggcggtg gctcgggcgg tggtgggtcg ggtggcggcg gatctgaggt 2940

gaaactgcag gagtcaggac ctggcctggt ggcgccctca cagagcctgt ccgtcacatg 3000

cactgtctca ggggtctcat tacccgacta tggtgtaagc tggattcgcc agcctccacg 3060

aaagggtctg gagtggctgg gagtaatatg gggtagtgaa accacatact ataattcagc 3120

tctcaaatcc agactgacca tcatcaagga caactccaag agccaagttt tcttaaaaat 3180

gaacagtctg caaactgatg acacagccat ttactactgt gccaaacatt attactacgg 3240

tggtagctat gctatggact actggggcca aggaacctca gtcaccgtct cctcaaccac 3300

gacgccagcg ccgcgaccac caacaccggc gcccaccatc gcgtcgcagc ccctgtccct 3360

gcgcccagag gcgtgccggc cagcggcggg gggcgcagtg cacacgaggg ggctggactt 3420

cgcctgtgat atctacatct gggcgccctt ggccgggact tgtggggtcc ttctcctgtc 3480

actggttatc accctttact gcaaacgggg cagaaagaaa ctcctgtata tattcaaaca 3540

accatttatg agaccagtac aaactactca agaggaagat ggctgtagct gccgatttcc 3600

agaagaagaa gaaggaggat gtgaactgag agtgaagttc agcaggagcg cagacgcccc 3660

cgcgtacaag cagggccaga accagctcta taacgagctc aatctaggac gaagagagga 3720

gtacgatgtt ttggacaaga gacgtggccg ggaccctgag atggggggaa agccgagaag 3780

gaagaaccct caggaaggcc tgtacaatga actgcagaaa gataagatgg cggaggccta 3840

cagtgagatt gggatgaaag gcgagcgccg gaggggcaag gggcacgatg gcctttacca 3900

gggtctcagt acagccacca aggacaccta cgacgccctt cacatgcagg ccctgccccc 3960

tcgctaaaca tgtttaaggg ttccggttcc actaggtaca attcgatatc aagcttatcg 4020

ataatcaacc tctggattac aaaatttgtg aaagattgac tggtattctt aactatgttg 4080

ctccttttac gctatgtgga tacgctgctt taatgccttt gtatcatgct attgcttccc 4140

gtatggcttt cattttctcc tccttgtata aatcctggtt gctgtctctt tatgaggagt 4200

tgtggcccgt tgtcaggcaa cgtggcgtgg tgtgcactgt gtttgctgac gcaaccccca 4260

ctggttgggg cattgccacc acctgtcagc tcctttccgg gactttcgct ttccccctcc 4320

ctattgccac ggcggaactc atcgccgcct gccttgcccg ctgctggaca ggggctcggc 4380

tgttgggcac tgacaattcc gtggtgttgt cggggaaatc atcgtccttt ccttggctgc 4440

tcgcctgtgt tgccacctgg attctgcgcg ggacgtcctt ctgctacgtc ccttcggccc 4500

tcaatccagc ggaccttcct tcccgcggcc tgctgccggc tctgcggcct cttccgcgtc 4560

ttcgccttcg ccctcagacg agtcggatct ccctttgggc cgcctccccg catcgatacc 4620

gtcgacctcg atcgagacct agaaaaacat ggagcaatca caagtagcaa tacagcagct 4680

accaatgctg attgtgcctg gctagaagca caagaggagg aggaggtggg ttttccagtc 4740

acacctcagg tacctttaag accaatgact tacaaggcag ctgtagatct tagccacttt 4800

ttaaaagaaa aggggggact ggaagggcta attcactccc aacgaagaca agatatcctt 4860

gatctgtgga tctaccacac acaaggctac ttccctgatt ggcagaacta cacaccaggg 4920

ccagggatca gatatccact gacctttgga tggtgctaca agctagtacc agttgagcaa 4980

gagaaggtag aagaagccaa tgaaggagag aacacccgct tgttacaccc tgtgagcctg 5040

catgggatgg atgacccgga gagagaagta ttagagtgga ggtttgacag ccgcctagca 5100

tttcatcaca tggcccgaga gctgcatccg gactgtactg ggtctctctg gttagaccag 5160

atctgagcct gggagctctc tggctaacta gggaacccac tgcttaagcc tcaataaagc 5220

ttgccttgag tgcttcaagt agtgtgtgcc cgtctgttgt gtgactctgg taactagaga 5280

tccctcagac ccttttagtc agtgtggaaa atctctagca 5320

<210> 2
<211> 4417
<212> DNA
<213> Artificial Sequence

<220>
<223> pPVLV2 vector containing CAR-19 coding sequence

<400> 2
gggtctctct ggttagacca gatctgagcc tgggagctct ctggctaact agggaaccca 60

ctgcttaagc ctcaataaag cttgccttga gtgcttcaag tagtgtgtgc ccgtctgttg 120

tgtgactctg gtaactagag atccctcaga cccttttagt cagtgtggaa aatctctagc 180

agtggcgccc gaacagggac ttgaaagcga aagggaaacc agaggagctc tctcgacgca 240

ggactcggct tgctgaagcg cgcacggcaa gaggcgaggg gcggcgactg gtgagtacgc 300

caaaaatttt gactagcgga ggctagaagg agagagatgg gtgcgagagc gtcagtatta 360

agcgggggag aattagatcg cgatgggaaa aaattcggtt aaggccaggg ggaaagaaaa 420

aatataaatt aaaacatata gtatgggcaa gcagggagct agaacgattc gcagttaatc 480

ctggcctgtt agaaacatca gaaggctgta gacaaatact gggacagcta caaccatccc 540

ttcagacagg atcagaagaa cttagatcat tatataatac agtagcaacc ctctattgtg 600

tgcatcaaag gatagagata aaagacacca aggaagcttt agacaagata gaggaagagc 660

aaaacaaaag taagaccacc gcacagcaag cggccgctga tcttcagacc tggaggagga 720

gatatgaggg acaattggag aagtgaatta tataaatata aagtagtaaa aattgaacca 780

ttaggagtag cacccaccaa ggcaaagaga agagtggtgc agagagaaaa aagagcagtg 840

ggaataggag ctttgttcct tgggttcttg ggagcagcag gaagcactat gggcgcagcg 900

tcaatgacgc tgacggtaca ggccagacaa ttattgtctg gtatagtgca gcagcagaac 960

aatttgctga gggctattga ggcgcaacag catctgttgc aactcacagt ctggggcatc 1020

aagcagctcc aggcaagaat cctggctgtg gaaagatacc taaaggatca acagctcctg 1080

gggatttggg gttgctctgg aaaactcatt tgcaccactg ctgtgccttg gaatgctagt 1140

tggagtaata aatctctgga acagatttgg aatcacacga cctggatgga gtgggacaga 1200

gaaattaaca attacacaag cttaatacac tccttaattg aagaatcgca aaaccagcaa 1260

gaaaagaatg aacaagaatt attggaatta gataaatggg caagtttgtg gaattggttt 1320

aacataacaa attggctgtg gtatataaaa ttattcataa tgatagtagg aggcttggta 1380

ggtttaagaa tagtttttgc tgtactttct atagtgaata gagttaggca gggatattca 1440

ccattatcgt ttcagaccca cctcccaacc ccgaggggac ccgacaggcc cgaaggaata 1500

gaagaagaag gtggagagag agacagagac agatccattc gattagtgaa cggatcggca 1560

ctgcgtgcgc caattctgca gacaaatggc agtattcatc cacaatttta aaagaaaagg 1620

ggggattggg gggtacagtg caggggaaag aatagtagac ataatagcaa cagacataca 1680

aactaaagaa ttacaaaaac aaattacaaa aattcaaaat tttcgggttt attacaggga 1740

cagcagagat ccagtttggt taaattcgct agctaggtct tgaaaggagt gggaattggc 1800

tccggtgccc gtcagtgggc agagcgcaca tcgcccacag tccccgagaa gttgggggga 1860

ggggtcggca attgatccgg tgcctagaga aggtggcgcg gggtaaactg ggaaagtgat 1920

gtcgtgtact ggctccgcct ttttcccgag ggtgggggag aaccgtatat aagtgcagta 1980

gtcgccgtga acgttctttt tcgcaacggg tttgccgcca gaacacagga ccggttctag 2040

aatggcctta ccagtgaccg ccttgctcct gccgctggcc ttgctgctcc acgccgccag 2100

gccggacatc cagatgacac agactacatc ctccctgtct gcctctctgg gagacagagt 2160

caccatcagt tgcagggcaa gtcaggacat tagtaaatat ttaaattggt atcagcagaa 2220

accagatgga actgttaaac tcctgatcta ccatacatca agattacact caggagtccc 2280

atcaaggttc agtggcagtg ggtctggaac agattattct ctcaccatta gcaacctgga 2340

gcaagaagat attgccactt acttttgcca acagggtaat acgcttccgt acacgttcgg 2400

aggggggacc aagctggaga tcacaggtgg cggtggctcg ggcggtggtg ggtcgggtgg 2460

cggcggatct gaggtgaaac tgcaggagtc aggacctggc ctggtggcgc cctcacagag 2520

cctgtccgtc acatgcactg tctcaggggt ctcattaccc gactatggtg taagctggat 2580

tcgccagcct ccacgaaagg gtctggagtg gctgggagta atatggggta gtgaaaccac 2640

atactataat tcagctctca aatccagact gaccatcatc aaggacaact ccaagagcca 2700

agttttctta aaaatgaaca gtctgcaaac tgatgacaca gccatttact actgtgccaa 2760

acattattac tacggtggta gctatgctat ggactactgg ggccaaggaa cctcagtcac 2820

cgtctcctca accacgacgc cagcgccgcg accaccaaca ccggcgccca ccatcgcgtc 2880

gcagcccctg tccctgcgcc cagaggcgtg ccggccagcg gcggggggcg cagtgcacac 2940

gagggggctg gacttcgcct gtgatatcta catctgggcg cccttggccg ggacttgtgg 3000

ggtccttctc ctgtcactgg ttatcaccct ttactgcaaa cggggcagaa agaaactcct 3060

gtatatattc aaacaaccat ttatgagacc agtacaaact actcaagagg aagatggctg 3120

tagctgccga tttccagaag aagaagaagg aggatgtgaa ctgagagtga agttcagcag 3180

gagcgcagac gcccccgcgt accagcaggg ccagaaccag ctctataacg agctcaatct 3240

aggacgaaga gaggagtacg atgttttgga caagagacgt ggccgggacc ctgagatggg 3300

gggaaagccg agaaggaaga accctcagga aggcctgtac aatgaactgc agaaagataa 3360

gatggcggag gcctacagtg agattgggat gaaaggcgag cgccggaggg gcaaggggca 3420

cgatggcctt taccagggtc tcagtacagc caccaaggac acctacgacg cccttcacat 3480

gcaggccctg ccccctcgct gaggatccac gcgttaagtc gacaatcaac ctctggatta 3540

caaaatttgt gaaagattga ctggtattct taactatgtt gctcctttta cgctatgtgg 3600

atacgctgct ttaatgcctt tgtatcatgc tattgcttcc cgtatggctt tcattttctc 3660

ctccttgtat aaatcctggt tgctgtctct ttatgaggag ttgtggcccg ttgtcaggca 3720

acgtggcgtg gtgtgcactg tgtttgctga cgcaaccccc actggttggg gcattgccac 3780

cacctgtcag ctcctttccg ggactttcgc tttccccctc cctattgcca cggcggaact 3840

catcgccgcc tgccttgccc gctgctggac aggggctcgg ctgttgggca ctgacaattc 3900

cgtggtgttg tcggggaaat catcgtcctt tccttggctg ctcgcctgtg ttgccacctg 3960

gattctgcgc gggacgtcct tctgctacgt cccttcggcc ctcaatccag cggaccttcc 4020

ttcccgcggc ctgctgccgg ctctgcggcc tcttccgcgt cttcgccttc gccctcagac 4080

gagtcggatc tccctttggg ccgcctcccc gcgtcgactt taagaccaat gacttacaag 4140

gcagctgtag atcttagcca ctttttaaaa gaaaaggggg gactggaagg gctaattcac 4200

tcccaacgaa gacaagatct gctttttgct tgtactgggt ctctctggtt agaccagatc 4260

tgagcctggg agctctctgg ctaactaggg aacccactgc ttaagcctca ataaagcttg 4320

ccttgagtgc ttcaagtagt gtgtgcccgt ctgttgtgtg actctggtaa ctagagatcc 4380

ctcagaccct tttagtcagt gtggaaaatc tctagca 4417

<210> 3
<211> 675
<212> DNA
<213> Artificial Sequence

<220>
<223> 5'HIV R-U5-??gag

<400> 3
gggtctctct ggttagacca gatctgagcc tgggagctct ctggctaact agggaaccca 60

ctgcttaagc ctcaataaag cttgccttga gtgcttcaag tagtgtgtgc ccgtctgttg 120

tgtgactctg gtaactagag atccctcaga cccttttagt cagtgtggaa aatctctagc 180

agtggcgccc gaacagggac ttgaaagcga aagggaaacc agaggagctc tctcgacgca 240

ggactcggct tgctgaagcg cgcacggcaa gaggcgaggg gcggcgactg gtgagtacgc 300

caaaaatttt gactagcgga ggctagaagg agagagatgg gtgcgagagc gtcagtatta 360

agcgggggag aattagatcg cgatgggaaa aaattcggtt aaggccaggg ggaaagaaaa 420

aatataaatt aaaacatata gtatgggcaa gcagggagct agaacgattc gcagttaatc 480

ctggcctgtt agaaacatca gaaggctgta gacaaatact gggacagcta caaccatccc 540

ttcagacagg atcagaagaa cttagatcat tatataatac agtagcaacc ctctattgtg 600

tgcatcaaag gatagagata aaagacacca aggaagcttt agacaagata gaggaagagc 660

aaaacaaaag taaga 675

<210> 4
<211> 858
<212> DNA
<213> Artificial Sequence

<220>
<223> HIV-psi (??)

<400> 4
gatcttcaga cctggaggag gagatatgag ggacaattgg agaagtgaat tatataaata 60

taaagtagta aaaattgaac cattaggagt agcacccacc aaggcaaaga gaagagtggt 120

gcagagagaa aaaagagcag tgggaatagg agctttgttc cttgggttct tgggagcagc 180

aggaagcact atgggcgcag cgtcaatgac gctgacggta caggccagac aattattgtc 240

tggtatagtg cagcagcaga acaatttgct gagggctatt gaggcgcaac agcatctgtt 300

gcaactcaca gtctggggca tcaagcagct ccaggcaaga atcctggctg tggaaagata 360

cctaaaggat caacagctcc tggggatttg gggttgctct ggaaaactca tttgcaccac 420

tgctgtgcct tggaatgcta gttggagtaa taaatctctg gaacagattt ggaatcacac 480

gacctggatg gagtgggaca gagaaattaa caattacaca agcttaatac actccttaat 540

tgaagaatcg caaaaccagc aagaaaagaa tgaacaagaa ttattggaat tagataaatg 600

ggcaagtttg tggaattggt ttaacataac aaattggctg tggtatataa aattattcat 660

aatgatagta ggaggcttgg taggtttaag aatagttttt gctgtacttt ctatagtgaa 720

tagagttagg cagggatatt caccattatc gtttcagacc cacctcccaa ccccgagggg 780

acccgacagg cccgaaggaa tagaagaaga aggtggagag agagacagag acagatccat 840

tcgattagtg aacggatc 858

<210> 5
<211> 234
<212> DNA
<213> Artificial Sequence

<220>
<223>  RRE

<400>  5
aggagctttg ttccttgggt tcttgggagc agcaggaagc actatgggcg cagcgtcaat      60

gacgctgacg gtacaggcca gacaattatt gtctggtata gtgcagcagc agaacaattt     120

gctgagggct attgaggcgc aacagcatct gttgcaactc acagtctggg gcatcaagca     180

gctccaggca agaatcctgg ctgtggaaag atacctaaag gatcaacagc tcct           234


<210>  6
<211>  118
<212>  DNA
<213>  Artificial Sequence

<220>
<223>  cPPT/CTS

<400>  6
ttttaaaaga aaagggggga ttggggggta cagtgcaggg gaaagaatag tagacataat      60

agcaacagac atacaaacta aagaattaca aaaacaaatt acaaaaattc aaaatttt       118


<210>  7
<211>  531
<212>  DNA
<213>  Artificial Sequence

<220>
<223>  EF1?? promoter long

<400>  7
tccggtgccc gtcagtgggc agagcgcaca tcgcccacag tccccgagaa gttgggggga      60

ggggtcggca attgaaccgg tgcctagaga aggtggcgcg gggtaaactg ggaaagtgat     120

gtcgtgtact ggctccgcct ttttcccgag ggtgggggag aaccgtatat aagtgcagta     180

gtcgccgtga acgttctttt tcgcaacggg tttgccgcca gaacacagct gaagcttcga     240

ggggctcgca tctctccttc acgcgcccgc cgccctacct gaggccgcca tccacgccgg     300

ttgagtcgcg ttctgccgcc tcccgcctgt ggtgcctcct gaactgcgtc cgccgtctag     360

gtaagtttaa agctcaggtc gagaccgggc ctttgtccgg cgctcccttg gagcctacct     420

agactcagcc ggctctccac gctttgcctg accctgcttg ctcaactcta cgtctttgtt     480

tcgttttctg ttctgcgccg ttacagatcc aagctgtgac cggcgcctac g              531


<210>  8
<211>  1458
<212>  DNA
<213>  Artificial Sequence

<220>
<223>  CAR-19 coding sequence

<400>  8
atggccttac cagtgaccgc cttgctcctg ccgctggcct tgctgctcca cgccgccagg      60

ccggacatcc agatgacaca gactacatcc tccctgtctg cctctctggg agacagagtc     120

accatcagtt gcagggcaag tcaggacatt agtaaatatt taaattggta tcagcagaaa     180

ccagatggaa ctgttaaact cctgatctac catacatcaa gattacactc aggagtccca     240

tcaaggttca gtggcagtgg gtctggaaca gattattctc tcaccattag caacctggag     300

caagaagata ttgccactta cttttgccaa cagggtaata cgcttccgta cacgttcgga     360

ggggggacca agctggagat cacaggtggc ggtggctcgg gcggtggtgg gtcgggtggc     420

ggcggatctg aggtgaaact gcaggagtca ggacctggcc tggtggcgcc ctcacagagc     480

ctgtccgtca catgcactgt ctcaggggtc tcattacccg actatggtgt aagctggatt     540

cgccagcctc cacgaaaggg tctggagtgg ctgggagtaa tatggggtag tgaaaccaca     600

tactataatt cagctctcaa atccagactg accatcatca aggacaactc caagagccaa     660

gttttcttaa aaatgaacag tctgcaaact gatgacacag ccatttacta ctgtgccaaa     720

cattattact acggtggtag ctatgctatg gactactggg gccaaggaac ctcagtcacc     780

gtctcctcaa ccacgacgcc agcgccgcga ccaccaacac cggcgcccac catcgcgtcg     840

cagcccctgt ccctgcgccc agaggcgtgc cggccagcgg cggggggcgc agtgcacacg     900

agggggctgg acttcgcctg tgatatctac atctgggcgc ccttggccgg gacttgtggg     960

gtccttctcc tgtcactggt tatcaccctt tactgcaaac ggggcagaaa gaaactcctg    1020

tatatattca aacaaccatt tatgagacca gtacaaacta ctcaagagga agatggctgt    1080

agctgccgat ttccagaaga agaagaagga ggatgtgaac tgagagtgaa gttcagcagg    1140

agcgcagacg cccccgcgta caagcagggc cagaaccagc tctataacga gctcaatcta    1200

ggacgaagag aggagtacga tgttttggac aagagacgtg gccgggaccc tgagatgggg    1260

ggaaagccga gaaggaagaa ccctcaggaa ggcctgtaca atgaactgca gaaagataag    1320

atggcggagg cctacagtga gattgggatg aaaggcgagc gccggagggg caaggggcac    1380

gatggccttt accagggtct cagtacagcc accaaggaca cctacgacgc ccttcacatg    1440

caggccctgc cccctcgc                                                  1458


<210>  9
<211>  590
<212>  DNA
<213>  Artificial Sequence

<220>
<223>  WPRE

<400>  9
taatcaacct ctggattaca aaatttgtga aagattgact ggtattctta actatgttgc      60

tccttttacg ctatgtggat acgctgcttt aatgcctttg tatcatgcta ttgcttcccg 120

tatggctttc attttctcct ccttgtataa atcctggttg ctgtctcttt atgaggagtt 180

gtggcccgtt gtcaggcaac gtggcgtggt gtgcactgtg tttgctgacg caacccccac 240

tggttggggc attgccacca cctgtcagct cctttccggg actttcgctt tccccctccc 300

tattgccacg gcggaactca tcgccgcctg ccttgcccgc tgctggacag gggctcggct 360

gttgggcact gacaattccg tggtgttgtc ggggaaatca tcgtcctttc cttggctgct 420

cgcctgtgtt gccacctgga ttctgcgcgg gacgtccttc tgctacgtcc cttcggccct 480

caatccagcg gaccttcctt cccgcggcct gctgccggct ctgcggcctc ttccgcgtct 540

tcgccttcgc cctcagacga gtcggatctc cctttgggcc gcctccccgc 590

<210> 10
<211> 690
<212> DNA
<213> Artificial Sequence

<220>
<223> 3′ SIN LTR

<400> 10
atcgagacct agaaaaacat ggagcaatca caagtagcaa tacagcagct accaatgctg 60

attgtgcctg gctagaagca caagaggagg aggaggtggg ttttccagtc acacctcagg 120

tacctttaag accaatgact tacaaggcag ctgtagatct tagccacttt ttaaaagaaa 180

aggggggact ggaagggcta attcactccc aacgaagaca agatatcctt gatctgtgga 240

tctaccacac acaaggctac ttccctgatt ggcagaacta cacaccaggg ccagggatca 300

gatatccact gacctttgga tggtgctaca agctagtacc agttgagcaa gagaaggtag 360

aagaagccaa tgaaggagag aacacccgct tgttacaccc tgtgagcctg catgggatgg 420

atgacccgga gagagaagta ttagagtgga ggtttgacag ccgcctagca tttcatcaca 480

tggcccgaga gctgcatccg gactgtactg ggtctctctg gttagaccag atctgagcct 540

gggagctctc tggctaacta gggaacccac tgcttaagcc tcaataaagc ttgccttgag 600

tgcttcaagt agtgtgtgcc cgtctgttgt gtgactctgg taactagaga tccctcagac 660

ccttttagtc agtgtggaaa atctctagca 690

<210> 11
<211> 181
<212> DNA
<213> Artificial Sequence

<220>
<223> truncated 5′LTR

<400> 11
gggtctctct ggttagacca gatctgagcc tgggagctct ctggctaact agggaaccca 60

ctgcttaagc ctcaataaag cttgccttga gtgcttcaag tagtgtgtgc ccgtctgttg 120

tgtgactctg gtaactagag atccctcaga cccttttagt cagtgtggaa aatctctagc 180

a 181

<210> 12
<211> 126
<212> DNA
<213> Artificial Sequence

<220>
<223> HIV-psi (??)

<400> 12
ctctctcgac gcaggactcg gcttgctgaa gcgcgcacgg caagaggcga ggggcggcga 60

ctggtgagta cgccaaaaat tttgactagc ggaggctaga aggagagaga tgggtgcgag 120

agcgtc 126

<210> 13
<211> 212
<212> DNA
<213> Artificial Sequence

<220>
<223> EF1?? promoter short

<400> 13
gggcagagcg cacatcgccc acagtccccg agaagttggg gggaggggtc ggcaattgat 60

ccggtgccta gagaaggtgg cgcggggtaa actgggaaag tgatgtcgtg tactggctcc 120

gcctttttcc cgagggtggg ggagaaccgt atataagtgc agtagtcgcc gtgaacgttc 180

tttttcgcaa cgggtttgcc gccagaacac ag 212

<210> 14
<211> 589
<212> DNA
<213> Artificial Sequence

<220>
<223> WPRE

<400> 14
aatcaacctc tggattacaa aatttgtgaa agattgactg gtattcttaa ctatgttgct 60

ccttttacgc tatgtggata cgctgcttta atgcctttgt atcatgctat tgcttcccgt 120

atggctttca ttttctcctc cttgtataaa tcctggttgc tgtctcttta tgaggagttg 180

tggcccgttg tcaggcaacg tggcgtggtg tgcactgtgt ttgctgacgc aacccccact 240

ggttggggca ttgccaccac ctgtcagctc ctttccggga ctttcgcttt ccccctccct 300

attgccacgg cggaactcat cgccgcctgc cttgcccgct gctggacagg ggctcggctg 360

ttgggcactg acaattccgt ggtgttgtcg gggaaatcat cgtcctttcc ttggctgctc 420

gcctgtgttg ccacctggat tctgcgcggg acgtccttct gctacgtccc ttcggccctc 480

aatccagcgg accttccttc ccgcggcctg ctgccggctc tgcggcctct tccgcgtctt 540

cgccttcgcc ctcagacgag tcggatctcc ctttgggccg cctccccgc 589

<210> 15
<211> 234
<212> DNA
<213> Artificial Sequence

<220>
<223> 3′ LTR ??-U3

<400> 15
tggaagggct aattcactcc caacgaagac aagatctgct ttttgcttgt actgggtctc 60

tctggttaga ccagatctga gcctgggagc tctctggcta actagggaac ccactgctta 120

agcctcaata aagcttgcct tgagtgcttc aagtagtgtg tgcccgtctg ttgtgtgact 180

ctggtaacta gagatccctc agaccctttt agtcagtgtg gaaaatctct agca 234

<210> 16
<211> 486
<212> PRT
<213> Artificial Sequence

<220>
<223> CAR-19 amino acid sequence

<400> 16

Met Ala Leu Pro Val Thr Ala Leu Leu Leu Pro Leu Ala Leu Leu Leu
1               5                   10                  15

His Ala Ala Arg Pro Asp Ile Gln Met Thr Gln Thr Thr Ser Ser Leu
            20                  25                  30

Ser Ala Ser Leu Gly Asp Arg Val Thr Ile Ser Cys Arg Ala Ser Gln
        35                  40                  45

Asp Ile Ser Lys Tyr Leu Asn Trp Tyr Gln Gln Lys Pro Asp Gly Thr
    50                  55                  60

Val Lys Leu Leu Ile Tyr His Thr Ser Arg Leu His Ser Gly Val Pro
65                  70                  75                  80

Ser Arg Phe Ser Gly Ser Gly Ser Gly Thr Asp Tyr Ser Leu Thr Ile
                85                  90                  95

Ser Asn Leu Glu Gln Glu Asp Ile Ala Thr Tyr Phe Cys Gln Gln Gly
            100                 105                 110

Asn Thr Leu Pro Tyr Thr Phe Gly Gly Gly Thr Lys Leu Glu Ile Thr
        115                 120                 125

Gly Gly Gly Gly Ser Gly Gly Gly Gly Ser Gly Gly Gly Gly Ser Glu
    130                 135                 140

Val Lys Leu Gln Glu Ser Gly Pro Gly Leu Val Ala Pro Ser Gln Ser
145                 150                 155                 160

Leu Ser Val Thr Cys Thr Val Ser Gly Val Ser Leu Pro Asp Tyr Gly
                165                 170                 175

Val Ser Trp Ile Arg Gln Pro Pro Arg Lys Gly Leu Glu Trp Leu Gly
            180                 185                 190

Val Ile Trp Gly Ser Glu Thr Thr Tyr Tyr Asn Ser Ala Leu Lys Ser
        195                 200                 205

Arg Leu Thr Ile Ile Lys Asp Asn Ser Lys Ser Gln Val Phe Leu Lys
    210                 215                 220

Met Asn Ser Leu Gln Thr Asp Asp Thr Ala Ile Tyr Tyr Cys Ala Lys
225                 230                 235                 240

His Tyr Tyr Tyr Gly Gly Ser Tyr Ala Met Asp Tyr Trp Gly Gln Gly
                245                 250                 255

Thr Ser Val Thr Val Ser Ser Thr Thr Thr Pro Ala Pro Arg Pro Pro
            260                 265                 270

Thr Pro Ala Pro Thr Ile Ala Ser Gln Pro Leu Ser Leu Arg Pro Glu
        275                 280                 285

Ala Cys Arg Pro Ala Ala Gly Gly Ala Val His Thr Arg Gly Leu Asp
    290                 295                 300

Phe Ala Cys Asp Ile Tyr Ile Trp Ala Pro Leu Ala Gly Thr Cys Gly
305                 310                 315                 320

Val Leu Leu Leu Ser Leu Val Ile Thr Leu Tyr Cys Lys Arg Gly Arg
                325                 330                 335

Lys Lys Leu Leu Tyr Ile Phe Lys Gln Pro Phe Met Arg Pro Val Gln
            340                 345                 350

Thr Thr Gln Glu Glu Asp Gly Cys Ser Cys Arg Phe Pro Glu Glu Glu

355                 360                 365

Glu Gly Gly Cys Glu Leu Arg Val Lys Phe Ser Arg Ser Ala Asp Ala
    370                 375                 380

Pro Ala Tyr Gln Gln Gly Gln Asn Gln Leu Tyr Asn Glu Leu Asn Leu
385                 390                 395                 400

Gly Arg Arg Glu Glu Tyr Asp Val Leu Asp Lys Arg Arg Gly Arg Asp
                405                 410                 415

Pro Glu Met Gly Gly Lys Pro Arg Arg Lys Asn Pro Gln Glu Gly Leu
            420                 425                 430

Tyr Asn Glu Leu Gln Lys Asp Lys Met Ala Glu Ala Tyr Ser Glu Ile
        435                 440                 445

Gly Met Lys Gly Glu Arg Arg Arg Gly Lys Gly His Asp Gly Leu Tyr
    450                 455                 460

Gln Gly Leu Ser Thr Ala Thr Lys Asp Thr Tyr Asp Ala Leu His Met
465                 470                 475                 480

Gln Ala Leu Pro Pro Arg
                485

<210>  17
<211>  219
<212>  PRT
<213>  Homo sapiens

<400>  17

Met Gly Arg Gly Leu Leu Arg Gly Leu Trp Pro Leu His Ile Val Leu
1               5                   10                  15

Trp Thr Arg Ile Ala Ser Thr Ile Pro Pro His Val Gln Lys Ser Val
            20                  25                  30

Asn Asn Asp Met Ile Val Thr Asp Asn Asn Gly Ala Val Lys Phe Pro
        35                  40                  45

Gln Leu Cys Lys Phe Cys Asp Val Arg Phe Ser Thr Cys Asp Asn Gln
    50                  55                  60

Lys Ser Cys Met Ser Asn Cys Ser Ile Thr Ser Ile Cys Glu Lys Pro
65                  70                  75                  80

Gln Glu Val Cys Val Ala Val Trp Arg Lys Asn Asp Glu Asn Ile Thr

85                  90                  95

Leu Glu Thr Val Cys His Asp Pro Lys Leu Pro Tyr His Asp Phe Ile
            100                 105                 110

Leu Glu Asp Ala Ala Ser Pro Lys Cys Ile Met Lys Glu Lys Lys Lys
        115                 120                 125

Pro Gly Glu Thr Phe Phe Met Cys Ser Cys Ser Ser Asp Glu Cys Asn
    130                 135                 140

Asp Asn Ile Ile Phe Ser Glu Glu Tyr Asn Thr Ser Asn Pro Asp Leu
145                 150                 155                 160

Leu Leu Val Ile Phe Gln Val Thr Gly Ile Ser Leu Leu Pro Pro Leu
                165                 170                 175

Gly Val Ala Ile Ser Val Ile Ile Ile Phe Tyr Cys Tyr Arg Val Asn
            180                 185                 190

Arg Gln Gln Lys Leu Ser Ser Thr Trp Glu Thr Gly Lys Thr Arg Lys
        195                 200                 205

Leu Met Glu Phe Ser Glu His Cys Ala Ile Ile
    210                 215

<210>  18
<211>  657
<212>  DNA
<213>  Homo sapiens

<400>  18
atgggtcggg ggctgctcag gggcctgtgg ccgctgcaca tcgtcctgtg gacgcgtatc      60

gccagcacga tcccaccgca cgttcagaag tcggttaata acgacatgat agtcactgac     120

aacaacggtg cagtcaagtt tccacaactg tgtaaatttt gtgatgtgag attttccacc     180

tgtgacaacc agaaatcctg catgagcaac tgcagcatca cctccatctg tgagaagcca     240

caggaagtct gtgtggctgt atggagaaag aatgacgaga acataacact agagacagtt     300

tgccatgacc ccaagctccc ctaccatgac tttattctgg aagatgctgc ttctccaaag     360

tgcattatga aggaaaaaaa aaagcctggt gagactttct tcatgtgttc ctgtagctct     420

gatgagtgca atgacaacat catcttctca gaagaatata acaccagcaa tcctgacttg     480

ttgctagtca tatttcaagt gacaggcatc agcctcctgc caccactggg agttgccata     540

tctgtcatca tcatcttcta ctgctaccgc gttaaccggc agcagaagct gagttcaacc     600

tgggaaaccg gcaagacgcg gaagctcatg gagttcagcg agcactgtgc catcatc        657

## Description

### Technical Field

The invention belongs to the field of biomedicine. Specifically, the present invention relates to improved t lentiviral vector, and preparation method and uses thereof. Specifically, the present invention relates to a lentiviral vector especially suitable for preparing a therapeutic T cell.

### Background

T cells are the key immune cells that kill tumor cells and virus-infected cells in the body. In recent years, T cells, including antigen-specific T cells derived from in vitro induced or tumor infiltrating lymphocytes, genetically modified chimeric antigen receptor T cells (CAR-T cells), and genetically modified T cell receptor T cells (TCR-T cells), have been used for the treatment of malignant tumors, showing significant tumor clearance and control effects in some clinical patients. However, due to the immune escape effect of tumor in patients, some tumor patients have resistance to the infused T cells, resulting in T cells not being able to exert their anti-tumor effects.

Both in vivo and in vitro studies have shown that TGF-β is an important T cell inhibitory factor, leading to the weakening or loss of the killing effect of T cells on target cells. Clinically, TGF-β is widely expressed in a variety of tumor tissues, and significantly inhibits the killing activity of tumor-specific T cells on tumor cells, which is an important reason for the failure of immunotherapy. The dominant negative TGF-β receptor type II (DNRII) is a negative regulatory receptor of TGF-β, which can inhibit the inhibitory effect of TGF-β on T cells. In animals, the killing effect of T cells on tumors can be significantly increased by administering or expressing T cell-specific DNRII, or administering soluble TGF-β RII, to interfere with the TGF-β signaling pathway. The research team led by Catherin M Bollard of Baylor College of Medicine found that giving patients EBV-specific T cells (EBV-CTL) treatment has a certain effect on Hodgkin and non-Hodgkin's lymphoma caused by EBV infection. However, in these diseases, the efficacy of EBV-CTL is disturbed due to the expression of TGF-β in tumor tissues. The research team used gene transduction to express DNRII on the surface of EBV-CTL cells for the treatment of relapsed Hodgkin's lymphoma. Among the 7 patients who can be evaluated, 4 patients achieved complete remission, of which 2 patients had complete remission lasting for 4 years, and one of them was the patient who failed to obtain complete remission after treatment with EBV-CTL without DNRII gene modification. However, these EBV-CTLs only express one exogenous protein, namely DNRII.

In the currently applied clinical treatment with CAR-T cells and TCR-T cells, the same issue remains that tumor cells express TGF-β which leads to the inhibition of CAR-T cells and TCR-T cell functions. It is desired in the art to introduce DNRII into CAR-T cells or TCR-T cells. However, so far, there has not been a report about the co-expression of CAR/TCR and DNRII in the same T cell for the treatment of tumors. This may be due to the low co-expression efficiency of the two proteins, which is difficult to meet clinical needs.

To solve this problem, there is a need of an improved expression vector, such as an improved lentiviral vector, which is especially suitable for co-expressing two or more proteins efficiently.

### Brief Description of Drawings

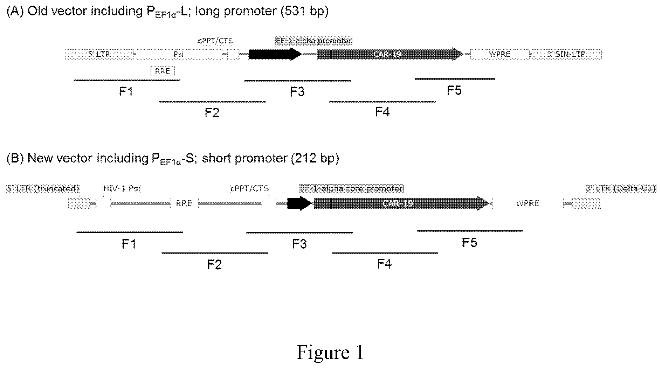

Figure 1 shows the genome of the lentiviral vector for expressing CAR-19 and the strategy for identifying integrity thereof. (A) The old vector pPVLV1 containing P_{EF1α}-L (long promoter, 531bp); (B) the new vector pPVLV2 containing P_{EF1α}-S (short promoter, 212bp). The integrity of the viral vector genome was identified by generating expected PCR products (F1-F5: PCR fragmenta) from cDNA reverse-transcribed using random hexamer primers.

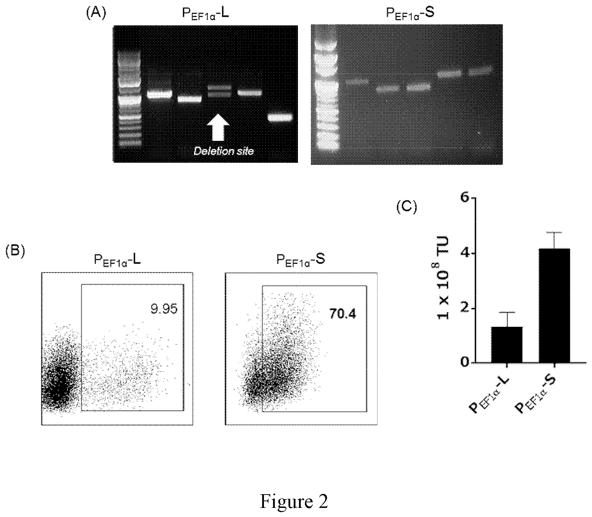

Figure 2 shows the difference between pPVLV1 and pPVLV2. (A) The expected DNA fragments were amplified from the reverse-transcribed cDNA of viral genome. Defective gene site was observed in the P_{EF1α}-L (long promoter) containing viral gene fragment. DNA fragment with unexpected size was indicated by arrows (left panel). (B) Comparison of the percentages of CAR-19 expressing cells, and (C) the titer of each vector 48 hours after transduction into 293T cells.

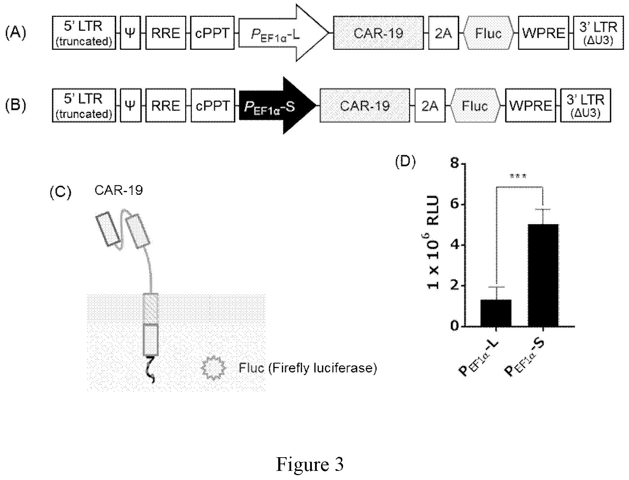

Figure 3 shows the structure and luciferase activity of the CAR-19-Fluc. (A) and (B) Bicistronic constructs encoding the CAR-19 cloned upstream of the P2A-Fluc cassette were used in this experiment. (C) Schematic representation of CAR-19 and Fluc molecules. (D) Luciferase activity of lentiviral vectors was determined 48 hrs after transduction of 293T cells.

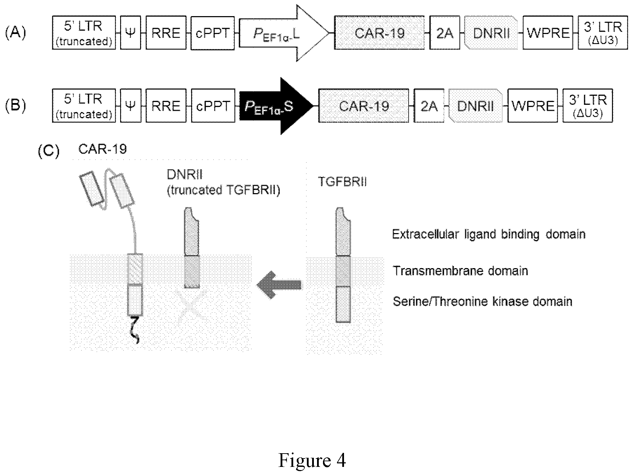

Figure 4 shows the structure and viral vector of CAR-19-DNRII. (A) and (B) show the vector map of CAR-19 co-expressing truncated TGFBRII (DNRII). (C) Schematic diagram of the co-expressed CAR-19 and DNRII molecules.

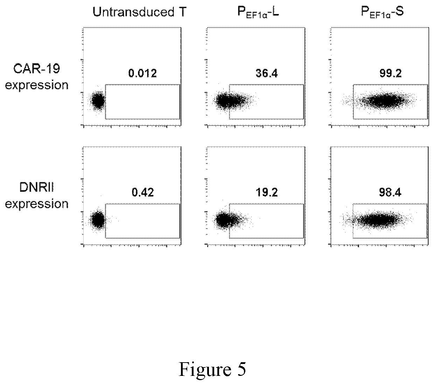

Figure 5 shows the transduction efficiency of CAR-19 and DNRII expression in transduced 293T cells. The numbers in the figure represent the percentage of CAR-19 (top) or DNRII (bottom) positive cells relative to the negative control of un-transduced 293T cells. The results of representative experiments from ten independent experiments are presented.

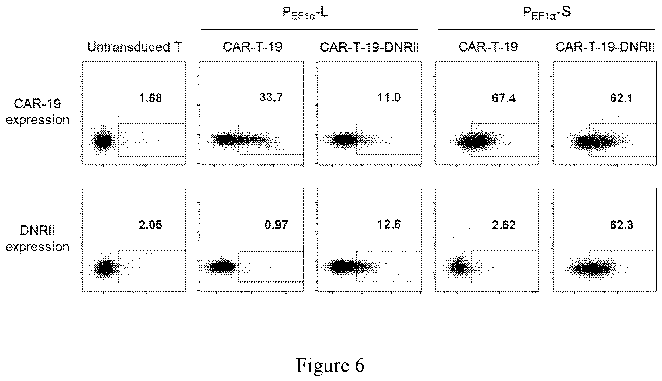

Figure 6 shows the expression of CAR-19 and DNRII in transduced T cells. The activated T cells were transduced with lentiviral vectors to express CAR-19 or CAR-19-DNRII, and evaluated by flow cytometry. The numbers in the figure represent the percentage of CAR-19 (top) or DNRII (bottom) positive cells relative to the negative control of un-transduced T cells. The results are representative of three independent experiments.

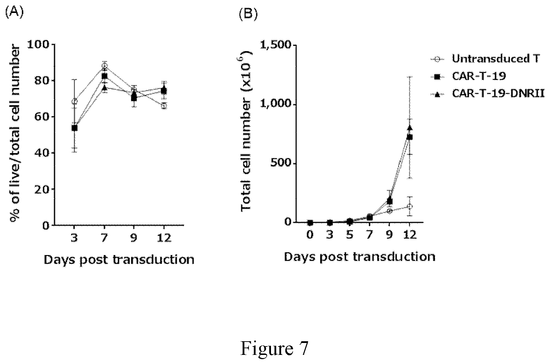

Figure 7 shows the cell viability and counts after transduction with CAR-19 or CAR-19-DNRII vector. Data are expressed as mean ± SD.

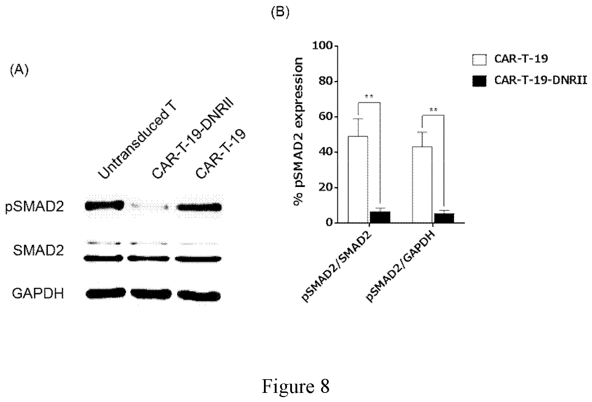

Figure 8 shows that DNRII reduced TGF-β1-induced phosphorylation of SMAD2.

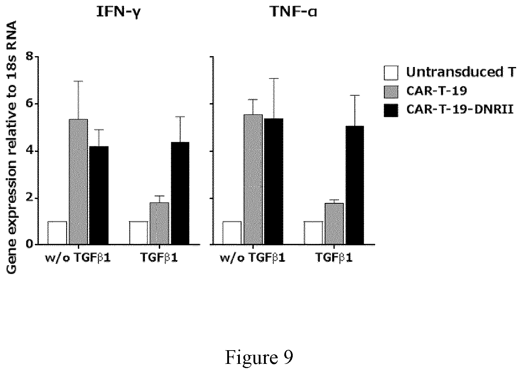

Figure 9 shows the mRNA levels of IFN-γ and TNF-α in CAR-T-19 and CAR-T-19-DNRII cells. Data are expressed as mean ± SEM

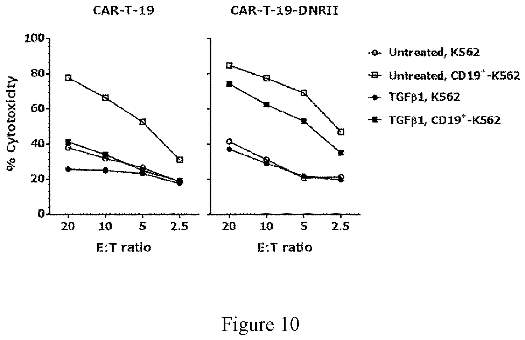

Figure 10 shows the antigen-specific killing of CD19+ tumor cells by CAR-T-19 and CAR-T-19-DNRII cells in the presence of TGF-β1. Twelve days after the initial activation of CAR T cells, the cell lysis activity was measured by the DELFIA® EuTDA cytotoxicity assay. T cells were collected 3 days before the measurement and cultured with rhTGF-β1 (10ng/ml) for 72 hours. The target cells were labeled with BATDA reagent for 15 minutes, and then transduced T cells as effector cells were added at the specified E:T ratio. Lysis was measured after 4 hours of incubation.

### Detailed Description of the Invention

Unless otherwise indicated or defined, all the terms used have their usual meanings in the art, which will be understood by those skilled in the art. Reference is made to, for example, standard manuals such as Sambrook et al., "Molecular Cloning: A Laboratory Manual"; Lewin, " Genes VIII"; and Roitt et al., " Immunology" (Version 8), and general prior art cited in this specification. In addition, unless otherwise described, all methods, steps, technologies, and operations that are not specifically detailed can be and have been performed in a manner known per se, which will be understood by those skilled in the art. Reference is also made to, for example, the standard manual, the above-mentioned general prior art and other references cited therein.

In a first aspect, the present invention provides a lentiviral vector comprising a truncated EF1α promoter for directing the expression of a nucleotide sequence encoding a polypeptide of interest in a host cell. In some embodiments, the truncated EF1α promoter is an EF1α core promoter comprising the nucleotide sequence setting forth in SEQ ID NO: 13.

Within the scope of the present invention, "lentiviral vector" refers to a non-replicating vector, which is used to transduce a transgene containing a cis-acting lentiviral RNA or DNA sequence to a host cell, where lentiviral proteins (for example, Gag, Pol and/or Env) need to be provided in trans form. Lentiviral vectors lack the coding sequences for functional Gag, Pol and Env proteins. Lentiviral vectors can exist in the form of RNA or DNA molecules, depending on the stage of production or development of the viral vector.

The lentiviral vector may be in the form of a recombinant DNA molecule, such as a plasmid (e.g., a transfer plasmid vector). The lentiviral vector may be in the form of a lentiviral particle vector, such as an RNA molecule in a complex of lentivirus and other proteins. Generally, a lentiviral vector corresponding to a modified or recombined lentiviral particle contains a genome composed of two copies of single-stranded RNA. These RNA sequences can be obtained by transcription from a double-stranded DNA sequence (proviral vector DNA) inserted into the genome of a host cell, or can be obtained by transient expression of plasmid DNA (plasmid vector DNA) in a transformed host cell. Lentiviral vector can also refer to a DNA sequence integrated into a host cell.

Lentiviral vector can be derived from lentiviruses, especially human immunodeficiency virus (HIV-1 or HIV-2), simian immunodeficiency virus (SIV), equine infectious encephalitis virus (EIAV), goat arthritis encephalitis virus (CAEV), bovine immunodeficiency virus (BIV) and feline immunodeficiency virus (FIV), which is modified to remove genetic determinants involved in pathogenicity and introduced with exogenous expression cassette.

In some embodiments, the lentiviral vector further comprises at least one element selected from the group consisting of a 5'LTR, a ψ sequence, an RRE sequence, a cPPT/CTS sequence, a multiple cloning site for inserting a nucleotide sequence encoding the polypeptide of interest, a WPRE sequence, and a 3'LTR.

In some embodiments, the lentiviral vector comprises a 5'LTR, a ψ element, an RRE element, a cPPT/CTS element, the truncated EF1α promoter, aWPRE component, a 3'LTR, and optionally, a multiple cloning site for inserting a nucleotide sequence encoding the polypeptide of interest, which are operably linked.

In some specific embodiments, the 5'LTR comprises the nucleotide sequence shown in SEQ ID NO: 3 or 11; the ψ element comprises the nucleotide sequence shown in SEQ ID NO: 4 or 12; the RRE element comprises the nucleotide sequence shown in SEQ ID NO: 5; the cPPT/CTS element comprises the nucleotide sequence shown in SEQ ID NO: 6; the WPRE element comprises the nucleotide sequence shown in SEQ ID NO: 9 or 14; the 3'LTR comprises the nucleotide sequence shown in SEQ ID NO: 10 or 15.

In some embodiments, the lentiviral vector comprises a 5'LTR comprising the nucleotide sequence shown in SEQ ID NO: 11, a ψ element comprising the nucleotide sequence shown in SEQ ID NO: 12, and an RRE element of the nucleotide sequence shown in SEQ ID NO: 5, a cPPT/CTS element including the nucleotide sequence shown in SEQ ID NO: 6, a truncated EF1α promoter of the nucleotide sequence shown in SEQ ID NO: 13, a WPRE element comprising the nucleotide sequence shown in SEQ ID NO: 14, a 3'LTR of the nucleotide sequence shown in SEQ ID NO: 15, and optionally, a multiple cloning site for inserting a nucleotide sequence encoding the polypeptide of interest, which are operably linked.

In some embodiments, the lentiviral vector is derived from SEQ ID NO: 2, wherein the nucleotide sequence from position 2,042 to position 3,499 of SEQ ID NO: 2 encoding CAR-19 can be replaced by a nucleotide sequence encoding t the polypeptide of interest.

In some embodiments, the lentiviral vector further comprises a nucleotide sequence encoding the polypeptide of interest.

In some embodiments, the polypeptide of interest is a fusion polypeptide comprising a plurality of proteins, the plurality of proteins in the fusion polypeptide being separated by a self-cleavable peptide.

In some embodiments, the polypeptide of interest is a fusion polypeptide comprising a first protein and a second protein, and the fusion polypeptide comprises a self-cleavable peptide between the first protein and the second protein.

Therefore, in some embodiments, the lentiviral vector further comprises a nucleotide sequence encoding a self-cleavable peptide. The coding nucleotide sequence of the self-cleavable peptide is used for co-expression of two or more different proteins by the lentiviral vector.

As used herein, "self-cleavable peptide" refers to a peptide that can achieve self-cleavage within a cell. For example, the self-cleavable peptide may include a protease recognition site, so that it can be recognized and specifically cleaved by the protease in the cell.

Alternatively, the self-cleaving peptide may be a 2A polypeptide. 2A polypeptide is a type of short peptides derived from viruses, and its self-cleavage occurs during translation. When 2A polypeptide is used to connect two different target proteins and expressed in the same reading frame, the two target proteins are almost produced at a ratio of 1:1. Commonly used 2A polypeptides can be P2A from porcine techovirus-1, T2A from Thosea asigna virus, and E2A from equine rhinitis A virus, and F2A from foot-and-mouth disease virus. Among them, P2A has the highest cutting efficiency and is therefore preferred. A variety of functional variants of these 2A polypeptides are also known in the art, and these variants can also be used in the present invention.

Separating the first protein and the second protein by the 2A polypeptide, placing them in a same open reading frame, and driving the expression by the same promoter, can maximize the possibility that the transduced cells express both proteins. Because if the two proteins are separately transduced into the cells in different vectors, some cells may only express the first protein, while some cells only express the second protein. The proportion of cells co-expressing the two proteins will be low. In addition, if the expression of two proteins is driven by different promoters in the same vector, due to the difference in promoter efficiency, the proportion of cells expressing the two proteins will also be reduced.

In some embodiments, the first protein is a cancer-associated antigen-specific receptor protein. In some embodiments, the second protein is a dominant negative TGF-β type II receptor.

As used in the present invention, "dominant negative TGF-β type II receptor" means a variant of the TGF-β type II receptor that can compete with TGF-β RII for binding to the TGF-β ligand (such as TGF-β1), but cannot perform TGF-β RII signal transduction function. In some embodiments, the intracellular signaling domain of the dominant negative TGF-β type II receptor is mutated, thereby losing the ability of intracellular signaling. In some embodiments, the dominant negative TGF-β type II receptor lacks the intracellular signaling domain of the TGF-β type II receptor. In some specific embodiments, the dominant negative TGF-β type II receptor comprises the amino acid sequence shown in SEQ ID NO:18.

The " cancer-associated antigen-specific receptor protein" of the present invention can be an exogenous T cell receptor (TCR) or a chimeric antigen receptor (CAR).

The cancer-associated antigen of the present invention includes but is not limited to CD16, CD64, CD78, CD96, CLL1, CD116, CD117, CD71, CD45, CD71, CD123, CD138, ErbB2 (HER2/neu), carcinoembryonic antigen (CEA), epithelial cell adhesion molecule (EpCAM), epidermal growth factor receptor (EGFR), EGFR variant III (EGFRvIII), CD19, CD20, CD30, CD40, disialylganglioside GD2, ductal epithelial mucin, gp36, TAG-72, glycosphingolipid, glioma-related antigens, β-human chorionic gonadotropin, α-fetoglobulin (AFP), lectin-responsive AFP, thyroglobulin, RAGE-1, MN-CAIX, human telomerase reverse transcriptase, RU1, RU2 (AS), intestinal carboxyl esterase, mut hsp70-2, M-CSF, prostase, prostatase specific antigen (PSA), PAP, NY-ESO-1, LAGA-1a, p53, Prostein, PSMA, survival and telomerase, prostate cancer tumor antigen-1 (PCTA-1), MAGE, ELF2M, neutrophil elastase, ephrin B2, CD22, insulin growth factor (IGF1)-I, IGF-II, IGFI receptor, mesothelin, major histocompatibility complex (MHC) molecules that present tumor-specific peptide epitopes, 5T4, ROR1, Nkp30, NKG2D, tumor stromal antigen, fibronectin extra domain A (EDA) and extra domain B (EDB), tenascin-C A1 domain (TnC A1), fibroblast-associated protein (fap), CD3, CD4, CD8, CD24 , CD25, CD33, CD34, CD133, CD138, Foxp3, B7-1 (CD80), B7-2 (CD86), GM-CSF, cytokine receptor, endothelial factor, BCMA (CD269, TNFRSF17), TNFRSF17 (UNIPROT Q02223), SLAMF7 (UNIPROT Q9NQ25), GPRC5D (UNIPROT Q9NZD1), FKBP11 (UNIPROT Q9NYL4), KAMP3, ITGA8 (UNIPROT P53708) and FCRL5 (UNIPROT Q68SN8).

"T cell receptor (TCR)", also known as T cell antigen receptor, is a molecular structure of T cell that specifically recognizes and binds antigen peptide-MHC molecules, and usually exists on the surface of T cell in the form of a complex with CD3 molecules. The TCR of most T cells is composed of α and β peptide chains, while the TCR of a few T cells is composed of γ and δ peptide chains.

"Chimeric antigen receptor (CAR)", also known as artificial T cell receptor, chimeric T cell receptor, or chimeric immune receptor, is an artificially designed receptor that can confer certain specificity to immune effector cells. Generally, this technology is used to confer T cells the ability to specifically recognize tumor surface antigens. In this way, a large number of targeting tumor killer cells can be produced.

The CAR may include an extracellular antigen binding domain against the cancer-associated antigen. The extracellular antigen binding domain may be, for example, a monoclonal antibody, a synthetic antibody, a human antibody, a humanized antibody, a single domain antibody, an antibody single-chain variable fragment (scFV), and an antigen-binding fragment thereof. For example, the extracellular antigen binding domain may be derived from one or more known antibodies including any commercially available antibody, such as FMC63, rituximab, alemtuzumab, epratuzumab, trastuzumab, bivatuzumab, cetuximab, labetuzumab, palivizumab, sevirumab, tuvirumab, basiliximab, daclizumab, infliximab, omalizumab, efalizumab, Keliximab, siplizumab, natalizumab, clenoliximab, pemtumomab, Edrecolomab, Cantuzumab, and the like.

In some embodiments of various aspects of the present invention, the CAR further includes a transmembrane domain and an intracellular signal transduction domain. The intracellular signal transduction domain of the CAR according to the present invention is responsible for the intracellular signal transduction after the extracellular ligand binding domain binds to the target, leading to the activation of immune cells and immune response. The intracellular signal transduction domain has the capability to activate at least one normal effector function of immune cells expressing the CAR. For example, the effector function of T cells may be cytolytic activity or auxiliary activity, including the secretion of cytokines.

The intracellular signal transduction domain of a CAR may be a cytoplasmic sequence, such as but not limited to the cytoplasmic sequence of T cell receptors and co-receptors (which act in concert to initiate signal transduction after antigen receptor binding), and any derivative or variant of these sequences and any synthetic sequence with the same functional capability. The Intracellular signal transduction domain includes two different types of cytoplasmic signal transduction sequences: the sequences that initiate antigen-dependent primary activation, and the sequences that act in an antigen-independent manner to provide secondary or co-stimulatory signals. The primary cytoplasmic signal transduction sequence may include a signal transduction motif referred to as the immunoreceptor tyrosine activation motif, ITAM. Non-limiting examples of the ITAM used in the present invention may include those derived from TCRζ, FcRγ, FcRβ, FcRε, CD3γ, CD3δ, CD3ε, CD5, CD22, CD79a, CD79b, and CD66d. In some embodiments, the intracellular signal transduction domain of the CAR may include the CD3ζ signal transduction domain. In some embodiments, the intracellular signal transduction domain of the CAR of the present invention further includes a costimulatory domain. In some embodiments, the costimulatory domain is selected from the 41BB costimulatory domain or the CD28 costimulatory domain.

CAR is expressed on the surface of cells. Therefore, the CAR may include a transmembrane domain. The suitable transmembrane domain of the CAR of the present invention has the following capabilities: (a) expression on the cell surface, preferably immune cells, such as but not limited to lymphocytes or natural killer (NK) cells, and (b) interacting with the ligand binding domain and intracellular signal transduction domain to guide the cellular response of immune cells to predetermined target cells. The transmembrane domain may be derived from natural or synthetic sources. The transmembrane domain may be derived from any membrane-binding protein or transmembrane protein. As a non-limiting example, the transmembrane domain may be derived from subunits of T cell receptors such as α subunits, β subunits, γ or δ subunits, polypeptides constituting the CD3 complex, and p55(α chain), p75 (β chain) or γ of IL-2 receptors, a subunit chain of Fc receptors, especially Fcγ receptor III or CD protein. Alternatively, the transmembrane domain may be synthetic, and may mainly include hydrophobic residues such as leucine and valine. In some embodiments, the transmembrane domain is derived from a human CD8 α chain. The transmembrane domain may further include a hinge region located between the extracellular ligand binding domain and the transmembrane domain. The hinge region is, for example, derived from the extracellular region of CD8, CD4 or CD28. In some embodiments, the hinge region is part of a human CD8 α chain.

In some specific embodiments of various aspects of the present invention, the CAR used in the present invention may include an extracellular antigen binding domain that specifically binds cancer-associated antigens (e.g., scFv), a CD8α hinge and a transmembrane domain, a CD3ζ signal transduction domain, and a 4-1BB costimulatory domain.

In some specific embodiments, the CAR comprises an extracellular antigen binding domain against CD19. In some specific embodiments, the CAR comprises the amino acid sequence shown in SEQ ID NO:16.

Co-expression of T cell receptor (TCR) or chimeric antigen receptor (CAR) and a dominant negative TGF-β type II receptor in T cells can relieve the inhibition of TGF-β on T cells and significantly increase the activity of TCR-T cells or CAR-T cells, such as tumor killing activity. As shown in the examples, the lentiviral vector of the present invention is particularly suitable for co-expression of T cell receptor (TCR) or chimeric antigen receptor (CAR) and the dominant negative TGF-β type II receptor in T cells.

In another aspect, the present invention provides a method for preparing a lentiviral vector particle, the method comprising:
a) co-transfect a suitable host cell with the lentiviral vector of the present invention, one or more packaging vectors expressing Gag and/or Pol, and an envelope vector expressing an envelope protein such as VSV-G;
b) culturing the transfected host cell to package the lentiviral vector into a lentiviral vector particle; and
c) harvesting the lentiviral vector particle produced in step b).

It is known in the art and those skilled in the art can easily obtain suitable one or more packaging vectors for expressing Gag and/or Pol, and envelope vectors for expressing envelope proteins such as VSV-G In some embodiments, the vector is a plasmid.

Suitable host cells for preparing lentiviral vector particles include but are not limited to 293T cells.

In another aspect, the present invention provides a lentiviral vector particle, which comprises the lentiviral vector of the present invention or is prepared by the above-mentioned method of the present invention.

In another aspect, the present invention provides the use of the lentiviral vector particle of the present invention in the preparation of a therapeutic T cell, wherein the therapeutic T cell expresses a cancer-related antigen-specific receptor protein, such as a T cell receptor (TCR) or a chimeric antigen receptor (CAR), and optionally a dominant negative TGF-β type II receptor.

In another aspect, the present invention provides a method for preparing a therapeutic T cell, which includes transducing the T cell with the lentiviral vector particle of the present invention. The transduction of the lentiviral vector particle will cause the therapeutic T cell to express the cancer-related antigen-specific receptor protein, such as a T cell receptor (TCR) or chimeric antigen receptor (CAR), and optionally a dominant negative TGF-β type II receptor.

The T cells of the present invention can be obtained from many non-limiting sources by various non-limiting methods, including peripheral blood mononuclear cells, bone marrow, lymph node tissues, umbilical cord blood, thymus tissues, ascites, pleural effusions, spleen tissues and tumors. In some embodiments, cell lines available and known to those skilled in the art can be used. In some embodiments, the cells may be derived from a healthy donor or from a patient diagnosed with cancer. In some embodiments, the cells may be part of a mixed population of cells exhibiting different phenotypic characteristics. For example, the T cells can be obtained by isolating peripheral blood mononuclear cells (PBMC), then activating and expanding by using specific antibodies.

In some embodiments of various aspects of the present invention, the T cells are derived from autologous cells of the subject. As used herein, "autologous" refers to that cells, cell lines, or cell populations used to treat the subject are derived from the subject. In some embodiments, the T cells are derived from allogeneic cells, such as from a donor compatible with the subject's human leukocyte antigen (HLA). Standard schemes can be used to convert cells from a donor into non-alloreactive cells and to replicate the cells as required, generating cells that can be administered to one or more patients.

In another aspect, the present invention provides a kit for producing a lentiviral vector particle, which comprises the lentiviral vector of the present invention, a suitable packaging vector, a suitable envelope vector and/or a suitable host cell such as 293T cell. The kit may also include a cell transfection reagent. In another aspect, the present invention provides a kit for expressing a polypeptide of interest in a cell, which comprises the lentiviral vector particle of the present invention.

### Examples

Statistical analysis in the examples was performed using GraphPad software (GraphPad Prism v5.0; GraphPad Software, San Diego, CA, USA). Data were analyzed by Paired t-test followed by the Newman-Keuls test. Results were expressed as the mean ± SEM. A p-value of <0.05 was considered significant.

### Example 1. Optimization of lentiviral vector for expression of CAR

The lentiviral vector used to transduce CAR should contain the required CAR transgene and be able to express CAR in the cell. Two third-generation lentiviral vectors for expressing CAR were designed, namely the old vector pPVLV1 (Figure 1A) and the new vector pPVLV2 (Figure 1B). pPVLV1 contains a 531 bp long human elongation factor 1α(EF1α) promoter, and pPVLV2 contains a 212 bp truncated human EF1α promoter. The various elements contained in the two vectors and their descriptions are shown in Table 1 below.

The CAR to be expressed in the examples of the application includes the scFv targeting CD19, the hinge and transmembrane domain of human CD8, the intracellular domain 4-1BB and CD3ζ. The amino acid of the CAR targeting CD19 is shown in SEQ ID NO: 16, and the nucleotide sequence is shown in SEQ ID NO: 8.

**Table 1. Related elements on pPVLV1 and pPVLV2 lentiviral vectors**

| Feature | Location (size, bp) | | description |
|---|---|---|---|
| | pPVLV1¹⁾ | pPVLV2²⁾ | |
| 5'LTR | 1-675 | 1-181 | Truncated 5' LTR from HIV-1. Essential for viral transcription, reverse transcription, and integration |
| | (675) | (181) | |
| | 5'HIV R-U5-Δ gag | truncated | |
| | (SEQ ID NO:3) | (SEQ ID NO:11) | |
| HIV-psi (ψ) | 703-1,560 | 228-353 | Packaging signal of HIV-1. Essential for transfer plasmid packaging. |
| | (858) | (126) | |
| | (SEQ ID NO:4) | (SEQ ID NO:12) | |
| RRE | 850-1,083 | 846-1,079 | Essential for Rev-dependent mRNA export from the nucleus to the cytoplasm of viral transcripts. |
| | (234) | (234) | |
| | (SEQ ID NO:5) | (SEQ ID NO:5) | |
| cPPT/CTS | 1,610,-1,727 | 1,606-1,723 | cPPT/CTS of HIV-1. Improves vector integration and transduction efficiency. |
| | (118) | (118) | |
| | (SEQ ID NO:6) | (SEQ ID NO:6) | |
| EF1α 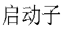 | 1,827-2,357 | 1,817-2,028 | Promoter that drives ubiquitous expression of the transgenes. |
| | (531) | (212) | |
| | (SEQ ID NO:7) | (SEQ ID NO:13) | |
| CAR-19 | 2,507-3,964 | 2,042-3,499 | transgene; CD 19 targeting chimeric antigen receptor. |
| | (1,458) | (1,458) | |
| | (SEQ ID NO:8) | (SEQ ID NO:8) | |
| WPRE | 4,022-4,611 | 3,524-4,112 | Improves transgene expression by facilitating mRNA transcript maturation. |
| | (590) | (589) | |
| | (SEQ ID NO:9) | (SEQ ID NO:14) | |
| 3' LTR | 4,631-5,320 | 4,184-4,417 | self-inactivating 3' LTR from HIV-1. Essential for viral transcription, reverse transcription and integration. Contains a safety measure to prevent viral replication. |
| | (690) | (234) | |
| | 3' SIN LTR | Δ-U3 | |
| | (SEQ ID NO:10) | (SEQ ID NO:15) | |

| | | | |
|---|---|---|---|
| 1) PPVLV1 vector including EF1α long promoter (5,320 bp, SEQ ID NO:1) 2) PPVLV2 vector including EF1α short promoter (4,417 bp, SEQ ID NO:2) | | | |

Lentiviral supernatant was created through transfection of 293T cells with gag/pol packaging plasmid, VSV-G envelope plasmid, and the transfer construct comprising the above-mentioned lentiviral vector sequences. Briefly, DNA mixtures were mixed in Opti-MEM (Life Technologies, Gaithersburg, MD, USA) and combined with equal volume of Opti-MEM containing Lipofectamine 3000 (Life Technologies). The resulting mixture was applied to 293T cells after 15 mins incubation at room temperature. Lentivirus-containing medium was collected at 24 hours post-transfection. After each collection, the supernatant was filtered through PVDF membrane (0.45 µm pore). Lentivirus harvests were combined and stored at 4°C before ultracentrifugation for 1 hour 30 mins at 20,000 x g. Lentiviral pellets were re-suspended in PBS.

Figure 1 shows a schematic diagram of the structure of two lentiviral vectors and a strategy for checking the integrity of the lentivirus by overlapping PCR products. Appropriate primers were designed to amplify overlapping fragments F1-F5 from cDNA reverse-transcribed using random primers. The PCR product with the expected size can prove the integrity of the lentivirus.

Figure 2A shows each DNA fragment amplified from cDNA reverse-transcribed from the viral genome. Unexpectedly, defective gene sites were observed in viral gene fragments containing P_{EF1α}-L (long promoter). The arrow indicates the presence of unexpected DNA fragments (left). This phenomenon was not observed in viral gene fragments containing P_{EF1α}-S (short promoter). Such defective viral genome may affect the titer and transduction efficiency.

To this end, the titers and transduction efficiency of the two lentiviruses were tested. For lentivirus titration, 2 x 10⁶ 293T cells were plated into each well of a 6-well plate and transduced with a range of volumes of the concentrated lentivirus. After 48 hours post-transduction, 293T cells were detached from plate. The presence of the CAR was detected through flow cytometry using a Alexa Fluor 488-labeled goat anti-human IgG F(ab)₂. Viral genomic RNA from 5' LTR to 3' LTR was checked using conventional PCR.

The results are shown in Figures 2B and C. The transduction efficiency (proportion of CAR-expressing cells) of the virus with P_{EF1α}-L (based on the pPVLV1 vector) is only 9.95%, which is much lower than the 70.4% of the virus with P_{EF1α}-S (based on the pPVLV2 vector). In addition, the titer of the virus with P_{EF1α}-L (based on the pPVLV1 vector) is also significantly lower than the lentivirus with P_{EF1α}-S (based on the pPVLV2 vector). It shows that pPVLV2 vector is better than pPVLV1 vector, and this may be caused by the different length of EF1α promoter.

### Example 2. The promoter affects the transduction and expression of CAR gene

In order to further prove the influence of different promoters, two CAR-luciferase reporter vectors shown in Figure 3 were constructed based on pPVLV2. The difference is only in the promoters for driving transgene expression, in which CAR-19 was cloned upstream of the P2A-Fluc (Firefly Fluorescence) cassette, thereby a bicistron is formed (Figure 3A and B).

After the vectors are transduced into the cell, due to the presence of the coding sequence of the P2A self-cleavable peptide, two molecules, CAR-19 and luciferase, will be expressed in the same cell at a ratio of approximately 1:1, where the fluorescence intensity can reflect the transduction efficiency of CAR-19 (see schematic diagram in Figure 3C). Figure 3D shows the luciferase activity measured 48 hours post transduction of the two lentiviral vectors into 293T cells. The results showed that the fluorescence of cells transduced with the lentiviral vector with P_{EF1α}-S was significantly stronger than that of the cells transduced with the lentiviral vector with P_{EF1α}-L, It is further proved that P_{EF1α}-S significantly improved the expression of transgene in cells.

This example proves that the conventional strong promoter, the 531bp EF1α promoter, when used for protein expression in a lentiviral vector, will unexpectedly lead to low transduction efficiency. By using the truncated EF1α promoter (212bp), the transduction efficiency can be significantly improved, and the expression of foreign proteins such as CAR in cells can be improved.

### Example 3. Co-expression of CAR and DNRII in cells

TGF-β is an important T cell inhibitory factor, which may lead to the weakening or loss of the killing effect of therapeutic T cells on target cells. Clinically, TGF-β is widely expressed in a variety of tumor tissues, and significantly inhibits the killing activity of tumor-specific T cells on tumor cells, which is an important reason for the failure of immunotherapy. The dominant negative TGF-β receptor type II (DNRII) is the negative regulatory receptor of TGF-β, which can inhibit the inhibitory effect of TGF-β on T cells. The following examples study the effect of co-expression of CAR and DNRII in T cells. The amino acid sequence of DNRII is shown in SEQ ID NO: 17, and its nucleotide sequence is shown in SEQ ID NO: 18.

First, similar to Example 2, wo CAR-19-DNRII vectors as shown in Figure 4 (A and B) were constructed based on pPVLV2. The difference is only in the promoters driving the expression of the transgenes. CAR-19 and DNRII were in the same open reading frame, with the 2A polypeptide coding sequence therebetween. Figure 4C shows a schematic diagram of the structure of CAR-19 and DNRII molecules. DNRII lacks the intracellular serine/threonine kinase domain of TGFBRII and cannot transmit signals downstream.

The CAR-19 and DNRII coding sequences are separated by the 2A coding sequence, placed in the same open reading frame, and expressed by the same promoter, which can ensure that the obtained transduced cells express both CAR-19 and DNRII. This is because if CAR-19 and DNRII are separately transduced into cells in different vectors, some cells may only express CAR-19 and some cells only express DNRII, and the proportion of cells co-expressing the two proteins will be very low. In addition, if the expression of two proteins is driven by different promoters in the same vector, due to the difference in promoter efficiency, the proportion of cells co-expressing both two proteins will also be reduced.

Two CAR-19-DNRII lentiviral vectors were transduced into 293T cells with equal MOI (multiplicity of infection). The expression of CAR or DNRII was detected with labeled goat anti-human IgG F(ab)₂ or anti-DNRII antibody by flow cytometry using MACSQuant analyzer 10, and the data was analyzed with FlowJo software.

The results are shown in Fig. 5. The expression of CAR-19 and DNRII in 293T cells transduced with the lentiviral vector with P_{EF1α}-S was significantly higher than that in 293T cells transduced with tje lentiviral vector with P_{EF1α}-L.

In addition, two CAR-19 lentiviral vectors and two CAR-19-DNRII lentiviral vectors were tested for the expression of CAR-19 and DNRII after transduction into T cells.

Human peripheral blood mononuclear cells (PBMC) from healthy donors were activated with anti-CD3/CD28 Dynabeads magnetic beads for 2 days (beads: cells=3:1), and resuspended at 1 × 10⁶ cells/ml supplemented with rhIL- 2 (200IU/mL) in IMSF100 serum-free medium (LONZA, Belgium). CAR-19 and CAR-19-DNRII lentiviral supernatants were added respectively for transduction, then centrifuged at 1,200×g at 32°C for 2 hours. After 24 hours, the supernatant containing the viral vector was removed. The cells were suspended in a medium containing rhIL-2 (200IU/mL) at 3×10⁵ cells/ml, and expanded and cultured with medium replacing every 2 to 3 days for 12 days to obtain CAR T-19 cells expressing CAR-19 molecules and CAR-T-19-DNRII cells co-expressing CAR-19 molecules and DNRII molecules. PBMCs cultured under the same culture conditions but not transduced were used as controls (NC). Flow cytometry was used to detect the expression of each protein molecule of the CAR-T cells obtained after transduction. Cells were stained with propidium iodide (PI) every 2-3 days, and cell viability was detected by flow cytometry. During the cell culture process, trypan blue staining was used to count the cells every 2-3 days (three replicates for each sample), and the number of cells was calculated (mean ± SD).

The results are shown in Figure 6, the non-transduced cells (NC) did not express CAR-19 or DNRII. CAR-T-19 using the pPVLV2 vector containing P_{EF1α}-S expressed CAR-19 (expression rate 67.4%); CAR-T-19-DNRII cells expressed both CAR-19 (expression rate 62.9%) and DNRII (the expression rate is 62.3%). It shows that CAR-19 and DNRII were co-expressed in CAR-T-19-DNRII cells, and the transduction efficiency is equivalent to that of CAR-19 alone. When using P_{EF1α}-L vector, CAR-19 and DNRII were also co-expressed in CAR-T-19-DNRII cells, but the expression rate was significantly reduced; while in CAR-T-19 cells, the expression rate of CAR-19 was also significant reduce.

In addition, as shown in Figure 7, there is no difference of cell viability and cell number between CAR-T-19-DNRII cells and CAR-T-19 cells.

Therefore, this example determined that the backbone of the pPVLV2 vector (comprsing P_{EF1α}-S) is particularly suitable for the expression of CAR in cells such as T cells, and is particularly suitable for co-expression of CAR and other proteins such as DNRII. In addition, placing CAR and DNRII coding sequences in the same open reading frame can achieve high co-expression rate of the two molecules.

### Example 4. The expression of DNRII reduces the phosphorylation of SMAD2 molecules induced by TFG-β1

The inhibitory effect of TFG-β on T cells is achieved by phosphorylation of SMAD2 molecules after TFG-β binds to its receptor.

After 9 days of transduction, CAR-T-19 cells and CAR-T-19-DNRII cells were incubated with recombinant human TFG-β1 (10ng/ml) for 24 hours to determine the expression level of phosphorylated SMAD2 (pSMAD2). With GAPDH and unphosphorylated SMAD2 molecules as controls, the relative quantification of pSMAD2 molecules was performed by western blot.

Specifically, protein concentrations of whole cell lysates were measured using a Bradford assay kit (Sigma-Aldrich). Equal amounts of protein were loaded into the wells of a SDS-PAGE gel and the separated proteins transferred to PVDF membranes (Thermo Scientific). The membranes were blocked with 10% (w/v) skim milk in TBST and then incubated with primary antibody (anti-pSMAD2 and -SMAD2 (Cell signaling Technologies, Danvers, MA, USA); all diluted 1:1000) overnight at 4°C. The membranes were then washed with TBST and incubated with an HRP-conjugated goat anti-rabbit IgG (diluted 1:2000; Cell Signaling Technologies) for 2 hours at room temperature. The membrane was then exposed to ECL reagents (Thermo Scientific) and the resulting signals detected using a Luminescent image analyzer (LAS-4000; Fuji Film, Tokyo, Japan)

The results are shown in Figure 8. The level of pSAMD2 in CAR-T-19-DNRII cells was significantly lower than that in CAR-T-19 cells. It shows that the expression of DNRII inhibits the phosphorylation of SMAD2, a key signaling molecule in the TGF-β signaling pathway.

### Example 5 Expression of IFN-γ and TNF-α in CAR-T-19-DNRII cells and CAR-T-19 cells treated with recombinant human TGF-β1

IFN-γ and TNF-α are the hallmark cytokines for T cells to kill target cells. The high expression levels of these two cytokines indicate that T cells have high killing potential to target cells, and vice versa.

Transduced-T cells were cultured with or without 10 ng/ml rhTGF-β1 for 24 hours following 9 days after post transduction. Then, each transduced-T cells were mixed CD19+-K562 for 24 hours, respectively. To determine the amounts of IFN-γ and TNF-α mRNA levels, each mixed cells were harvested and extracted the total RNA using PureLink RNA Mini kit (Thermo Scientific, Waltham, MA, USA). After DNase digestion and concentration determination using an Agilent 2100 Bioanalyzer (Agilent Technologies, Palo Alto, USA), total RNA samples were subjected to real-time quantitative RT-PCR analysis with specific primers and One-step SensiFAST SYBR Low-ROX kit (Bioline, Maryland, USA), using a QuantStudio3 Real-Time PCR detection system (Applied Biosystems, Foster City, CA, USA). The 18s rRNA was amplified as an internal control. Expression level was calculated by ΔΔCt method, and fold expression were obtained using the formula 2-ΔΔCt. All experiments were run in triplicate.

The results showed that after treatment with recombinant human TGF-β 1, the expression of IFN-γ and TNF-α in CAR-T-19-DNRII cells was significantly higher than that in CAR-T-19 cells (Figure 9).

### Example 6. Specifically killing of tumor target cells by CAR-T-19-DNRII cells and CAR-T-19 cells treated with recombinant human TGF-β1

Target cell killing experiments were performed using CAR-T-19 cells and CAR-T-19-DNRII cells 12 days post transduction .

TDA release assay was performed to determine the cytotoxic activity of CAR-T-19 cells and CAR-T-19-DNRII cells against K562 or CD19+-K562 in the presence of TGF-β1. CAR-T-19 cells and CAR-T-19-DNRII cells were incubated with recombinant human TGF-β1 (10ng/ml) for 72 hours. The target cells were labeled with BA-TDA (Perkin Elmer, Norwalk, Connecticut, USA) for 15 minutes, and mixed with effector cells according to the effector cell (T cell) : target cell (tumor cell) ratio of 20:1, 10:1, 5:1, and 2.5:1 respectively, and TDA release (target cell lysis) was detected after 4 hours of co-incubation. A time-resolved fluorescence (TRF) reader (Thermo Scientific) was used to detect the TDA release of the assay supernatant. The specific lysis is calculated as follows: % lysis = (experimental lysis-spontaneous lysis)/(maximum lysis-spontaneous lysis)×100.

The results are shown in Figure 10. After treatment with recombinant human TGF-β1, the killing effect of CAR-T-19 cells on K562 target cells expressing CD19 was reduced to the background (without CAR-T cells) level (Figure 10A). The killing effect of CAR-T-19-DNRII cells on K562 target cells expressing CD19 nearly did not decrease, which was significantly different from the killing effect without the addition of CAR-T cells (Figure 10B). It shows that DNRII effectively reversed the inhibitory effect of TGF-β on T cell killing.

### Sequence listing

SEQ ID NO:1 pPVLV1 vector containing CAR-19 coding sequence
GGGTCTCTCTGGTTAGACCAGATCTGAGCCTGGGAGCTCTCTGGCTAACTAGGGAACCCACTGCTTAAGCCTCAATAAAGCTT
GCCTTGAGTGCTTCAAGTAGTGTGTGCCCGTCTGTTGTGTGACTCTGGTAACTAGAGATCCCTCAGACCCTTTTAGTCAGTGT
GGAAAATCTCTAGCAGTGGCGCCCGAACAGGGACTTGAAAGCGAAAGGGAAACCAGAGGAGCTCTCTCGACGCAGGACTCGGC
TTGCTGAAGCGCGCACGGCAAGAGGCGAGGGGCGGCGACTGGTGAGTACGCCAAAAATTTTGACTAGCGGAGGCTAGAAGGAG
AGAGATGGGTGCGAGAGCGTCAGTATTAAGCGGGGGAGAATTAGATCGCGATGGGAAAAAATTCGGTTAAGGCCAGGGGGAAA
GAAAAAATATAAATTAAAACATATAGTATGGGCAAGCAGGGAGCTAGAACGATTCGCAGTTAATCCTGGCCTGTTAGAAACAT
CAGAAGGCTGTAGACAAATACTGGGACAGCTACAACCATCCCTTCAGACAGGATCAGAAGAACTTAGATCATTATATAATACA
GTAGCAACCCTCTATTGTGTGCATCAAAGGATAGAGATAAAAGACACCAAGGAAGCTTTAGACAAGATAGAGGAAGAGCAAAA
CAAAAGTAAGACCACCGCACAGCAAGCGGCCGGCCGCTGATCTTCAGACCTGGAGGAGGAGATATGAGGGACAATTGGAGAAG
TGAATTATATAAATATAAAGTAGTAAAAATTGAACCATTAGGAGTAGCACCCACCAAGGCAAAGAGAAGAGTGGTGCAGAGAG
AAAAAAGAGCAGTGGGAATAGGAGCTTTGTTCCTTGGGTTCTTGGGAGCAGCAGGAAGCACTATGGGCGCAGCGTCAATGACG
CTGACGGTACAGGCCAGACAATTATTGTCTGGTATAGTGCAGCAGCAGAACAATTTGCTGAGGGCTATTGAGGCGCAACAGCA
TCTGTTGCAACTCACAGTCTGGGGCATCAAGCAGCTCCAGGCAAGAATCCTGGCTGTGGAAAGATACCTAAAGGATCAACAGC
TCCTGGGGATTTGGGGTTGCTCTGGAAAACTCATTTGCACCACTGCTGTGCCTTGGAATGCTAGTTGGAGTAATAAATCTCTG
GAACAGATTTGGAATCACACGACCTGGATGGAGTGGGACAGAGAAATTAACAATTACACAAGCTTAATACACTCCTTAATTGA
AGAATCGCAAAACCAGCAAGAAAAGAATGAACAAGAATTATTGGAATTAGATAAATGGGCAAGTTTGTGGAATTGGTTTAACA
TAACAAATTGGCTGTGGTATATAAAATTATTCATAATGATAGTAGGAGGCTTGGTAGGTTTAAGAATAGTTTTTGCTGTACTT
TCTATAGTGAATAGAGTTAGGCAGGGATATTCACCATTATCGTTTCAGACCCACCTCCCAACCCCGAGGGGACCCGACAGGCC
CGAAGGAATAGAAGAAGAAGGTGGAGAGAGAGACAGAGACAGATCCATTCGATTAGTGAACGGATCGGCACTGCGTGCGCCAA
TTCTGCAGACAAATGGCAGTATTCATCCACAATTTTAAAAGAAAAGGGGGGATTGGGGGGTACAGTGCAGGGGAAAGAATAGT
AGACATAATAGCAACAGACATACAAACTAAAGAATTACAAAAACAAATTACAAAAATTCAAAATTTTCGGGTTTATTACAGGG
ACAGCAGAGATCCAGTTTGGTTAGTACCGGGCCCGACGTCGCATGCTCCCGGCCGCCATGGCGGCCGCGGGAATTCGATTAGA
TCCGGTGCCCGTCAGTGGGCAGAGCGCACATCGCCCACAGTCCCCGAGAAGTTGGGGGGAGGGGTCGGCAATTGAACCGGTGC
CTAGAGAAGGTGGCGCGGGGTAAACTGGGAAAGTGATGTCGTGTACTGGCTCCGCCTTTTTCCCGAGGGTGGGGGAGAACCGT
ATATAAGTGCAGTAGTCGCCGTGAACGTTCTTTTTCGCAACGGGTTTGCCGCCAGAACACAGCTGAAGCTTCGAGGGGCTCGC
ATCTCTCCTTCACGCGCCCGCCGCCCTACCTGAGGCCGCCATCCACGCCGGTTGAGTCGCGTTCTGCCGCCTCCCGCCTGTGG
TGCCTCCTGAACTGCGTCCGCCGTCTAGGTAAGTTTAAAGCTCAGGTCGAGACCGGGCCTTTGTCCGGCGCTCCCTTGGAGCC
TACCTAGACTCAGCCGGCTCTCCACGCTTTGCCTGACCCTGCTTGCTCAACTCTACGTCTTTGTTTCGTTTTCTGTTCTGCGC
CGTTACAGATCCAAGCTGTGACCGGCGCCTACGTAAGTGATATCTACTAGATTTATCAAAAAGAGTGTTGACTTGTGAGCGCT
CACAATTGATACTTAGATTCATCGAGAGGGACACGTCGACTACTAACCTTCTTCTCTTTCCTACAGCTGAGATCGCCGGTGGG
ATCCCCTAGGGTTAACATGGCCTTACCAGTGACCGCCTTGCTCCTGCCGCTGGCCTTGCTGCTCCACGCCGCCAGGCCGGACA
TCCAGATGACACAGACTACATCCTCCCTGTCTGCCTCTCTGGGAGACAGAGTCACCATCAGTTGCAGGGCAAGTCAGGACATT
AGTAAATATTTAAATTGGTATCAGCAGAAACCAGATGGAACTGTTAAACTCCTGATCTACCATACATCAAGATTACACTCAGG
AGTCCCATCAAGGTTCAGTGGCAGTGGGTCTGGAACAGATTATTCTCTCACCATTAGCAACCTGGAGCAAGAAGATATTGCCA
CTTACTTTTGCCAACAGGGTAATACGCTTCCGTACACGTTCGGAGGGGGGACCAAGCTGGAGATCACAGGTGGCGGTGGCTCG
GGCGGTGGTGGGTCGGGTGGCGGCGGATCTGAGGTGAAACTGCAGGAGTCAGGACCTGGCCTGGTGGCGCCCTCACAGAGCCT
GTCCGTCACATGCACTGTCTCAGGGGTCTCATTACCCGACTATGGTGTAAGCTGGATTCGCCAGCCTCCACGAAAGGGTCTGG
AGTGGCTGGGAGTAATATGGGGTAGTGAAACCACATACTATAATTCAGCTCTCAAATCCAGACTGACCATCATCAAGGACAAC
TCCAAGAGCCAAGTTTTCTTAAAAATGAACAGTCTGCAAACTGATGACACAGCCATTTACTACTGTGCCAAACATTATTACTA
CGGTGGTAGCTATGCTATGGACTACTGGGGCCAAGGAACCTCAGTCACCGTCTCCTCAACCACGACGCCAGCGCCGCGACCAC
CAACACCGGCGCCCACCATCGCGTCGCAGCCCCTGTCCCTGCGCCCAGAGGCGTGCCGGCCAGCGGCGGGGGGCGCAGTGCAC
ACGAGGGGGCTGGACTTCGCCTGTGATATCTACATCTGGGCGCCCTTGGCCGGGACTTGTGGGGTCCTTCTCCTGTCACTGGT
TATCACCCTTTACTGCAAACGGGGCAGAAAGAAACTCCTGTATATATTCAAACAACCATTTATGAGACCAGTACAAACTACTC
AAGAGGAAGATGGCTGTAGCTGCCGATTTCCAGAAGAAGAAGAAGGAGGATGTGAACTGAGAGTGAAGTTCAGCAGGAGCGCA
GACGCCCCCGCGTACAAGCAGGGCCAGAACCAGCTCTATAACGAGCTCAATCTAGGACGAAGAGAGGAGTACGATGTTTTGGA
CAAGAGACGTGGCCGGGACCCTGAGATGGGGGGAAAGCCGAGAAGGAAGAACCCTCAGGAAGGCCTGTACAATGAACTGCAGA
AAGATAAGATGGCGGAGGCCTACAGTGAGATTGGGATGAAAGGCGAGCGCCGGAGGGGCAAGGGGCACGATGGCCTTTACCAG
GGTCTCAGTACAGCCACCAAGGACACCTACGACGCCCTTCACATGCAGGCCCTGCCCCCTCGCTAAACATGTTTAAGGGTTCC
GGTTCCACTAGGTACAATTCGATATCAAGCTTATCGATAATCAACCTCTGGATTACAAAATTTGTGAAAGATTGACTGGTATT
CTTAACTATGTTGCTCCTTTTACGCTATGTGGATACGCTGCTTTAATGCCTTTGTATCATGCTATTGCTTCCCGTATGGCTTT
CATTTTCTCCTCCTTGTATAAATCCTGGTTGCTGTCTCTTTATGAGGAGTTGTGGCCCGTTGTCAGGCAACGTGGCGTGGTGT
GCACTGTGTTTGCTGACGCAACCCCCACTGGTTGGGGCATTGCCACCACCTGTCAGCTCCTTTCCGGGACTTTCGCTTTCCCC
CTCCCTATTGCCACGGCGGAACTCATCGCCGCCTGCCTTGCCCGCTGCTGGACAGGGGCTCGGCTGTTGGGCACTGACAATTC
CGTGGTGTTGTCGGGGAAATCATCGTCCTTTCCTTGGCTGCTCGCCTGTGTTGCCACCTGGATTCTGCGCGGGACGTCCTTCT
GCTACGTCCCTTCGGCCCTCAATCCAGCGGACCTTCCTTCCCGCGGCCTGCTGCCGGCTCTGCGGCCTCTTCCGCGTCTTCGC
CTTCGCCCTCAGACGAGTCGGATCTCCCTTTGGGCCGCCTCCCCGCATCGATACCGTCGACCTCGATCGAGACCTAGAAAAAC
ATGGAGCAATCACAAGTAGCAATACAGCAGCTACCAATGCTGATTGTGCCTGGCTAGAAGCACAAGAGGAGGAGGAGGTGGGT
TTTCCAGTCACACCTCAGGTACCTTTAAGACCAATGACTTACAAGGCAGCTGTAGATCTTAGCCACTTTTTAAAAGAAAAGGG
GGGACTGGAAGGGCTAATTCACTCCCAACGAAGACAAGATATCCTTGATCTGTGGATCTACCACACACAAGGCTACTTCCCTG
ATTGGCAGAACTACACACCAGGGCCAGGGATCAGATATCCACTGACCTTTGGATGGTGCTACAAGCTAGTACCAGTTGAGCAA
GAGAAGGTAGAAGAAGCCAATGAAGGAGAGAACACCCGCTTGTTACACCCTGTGAGCCTGCATGGGATGGATGACCCGGAGAG
AGAAGTATTAGAGTGGAGGTTTGACAGCCGCCTAGCATTTCATCACATGGCCCGAGAGCTGCATCCGGACTGTACTGGGTCTC
TCTGGTTAGACCAGATCTGAGCCTGGGAGCTCTCTGGCTAACTAGGGAACCCACTGCTTAAGCCTCAATAAAGCTTGCCTTGA
GTGCTTCAAGTAGTGTGTGCCCGTCTGTTGTGTGACTCTGGTAACTAGAGATCCCTCAGACCCTTTTAGTCAGTGTGGAAAAT
CTCTAGCA

SEQ ID NO:2    pPVLV2 vector containing CAR-19 coding sequence
GGGTCTCTCTGGTTAGACCAGATCTGAGCCTGGGAGCTCTCTGGCTAACTAGGGAACCCACTGCTTAAGCCTCAATAAAGCTT
GCCTTGAGTGCTTCAAGTAGTGTGTGCCCGTCTGTTGTGTGACTCTGGTAACTAGAGATCCCTCAGACCCTTTTAGTCAGTGT
GGAAAATCTCTAGCAGTGGCGCCCGAACAGGGACTTGAAAGCGAAAGGGAAACCAGAGGAGCTCTCTCGACGCAGGACTCGGC
TTGCTGAAGCGCGCACGGCAAGAGGCGAGGGGCGGCGACTGGTGAGTACGCCAAAAATTTTGACTAGCGGAGGCTAGAAGGAG
AGAGATGGGTGCGAGAGCGTCAGTATTAAGCGGGGGAGAATTAGATCGCGATGGGAAAAAATTCGGTTAAGGCCAGGGGGAAA
GAAAAAATATAAATTAAAACATATAGTATGGGCAAGCAGGGAGCTAGAACGATTCGCAGTTAATCCTGGCCTGTTAGAAACAT
CAGAAGGCTGTAGACAAATACTGGGACAGCTACAACCATCCCTTCAGACAGGATCAGAAGAACTTAGATCATTATATAATACA
GTAGCAACCCTCTATTGTGTGCATCAAAGGATAGAGATAAAAGACACCAAGGAAGCTTTAGACAAGATAGAGGAAGAGCAAAA
CAAAAGTAAGACCACCGCACAGCAAGCGGCCGCTGATCTTCAGACCTGGAGGAGGAGATATGAGGGACAATTGGAGAAGTGAA
TTATATAAATATAAAGTAGTAAAAATTGAACCATTAGGAGTAGCACCCACCAAGGCAAAGAGAAGAGTGGTGCAGAGAGAAAA
AAGAGCAGTGGGAATAGGAGCTTTGTTCCTTGGGTTCTTGGGAGCAGCAGGAAGCACTATGGGCGCAGCGTCAATGACGCTGA
CGGTACAGGCCAGACAATTATTGTCTGGTATAGTGCAGCAGCAGAACAATTTGCTGAGGGCTATTGAGGCGCAACAGCATCTG
TTGCAACTCACAGTCTGGGGCATCAAGCAGCTCCAGGCAAGAATCCTGGCTGTGGAAAGATACCTAAAGGATCAACAGCTCCT
GGGGATTTGGGGTTGCTCTGGAAAACTCATTTGCACCACTGCTGTGCCTTGGAATGCTAGTTGGAGTAATAAATCTCTGGAAC
AGATTTGGAATCACACGACCTGGATGGAGTGGGACAGAGAAATTAACAATTACACAAGCTTAATACACTCCTTAATTGAAGAA
TCGCAAAACCAGCAAGAAAAGAATGAACAAGAATTATTGGAATTAGATAAATGGGCAAGTTTGTGGAATTGGTTTAACATAAC
AAATTGGCTGTGGTATATAAAATTATTCATAATGATAGTAGGAGGCTTGGTAGGTTTAAGAATAGTTTTTGCTGTACTTTCTA
TAGTGAATAGAGTTAGGCAGGGATATTCACCATTATCGTTTCAGACCCACCTCCCAACCCCGAGGGGACCCGACAGGCCCGAA
GGAATAGAAGAAGAAGGTGGAGAGAGAGACAGAGACAGATCCATTCGATTAGTGAACGGATCGGCACTGCGTGCGCCAATTCT
GCAGACAAATGGCAGTATTCATCCACAATTTTAAAAGAAAAGGGGGGATTGGGGGGTACAGTGCAGGGGAAAGAATAGTAGAC
ATAATAGCAACAGACATACAAACTAAAGAATTACAAAAACAAATTACAAAAATTCAAAATTTTCGGGTTTATTACAGGGACAG
CAGAGATCCAGTTTGGTTAAATTCGCTAGCTAGGTCTTGAAAGGAGTGGGAATTGGCTCCGGTGCCCGTCAGTGGGCAGAGCG
CACATCGCCCACAGTCCCCGAGAAGTTGGGGGGAGGGGTCGGCAATTGATCCGGTGCCTAGAGAAGGTGGCGCGGGGTAAACT
GGGAAAGTGATGTCGTGTACTGGCTCCGCCTTTTTCCCGAGGGTGGGGGAGAACCGTATATAAGTGCAGTAGTCGCCGTGAAC
GTTCTTTTTCGCAACGGGTTTGCCGCCAGAACACAGGACCGGTTCTAGAATGGCCTTACCAGTGACCGCCTTGCTCCTGCCGC
TGGCCTTGCTGCTCCACGCCGCCAGGCCGGACATCCAGATGACACAGACTACATCCTCCCTGTCTGCCTCTCTGGGAGACAGA
GTCACCATCAGTTGCAGGGCAAGTCAGGACATTAGTAAATATTTAAATTGGTATCAGCAGAAACCAGATGGAACTGTTAAACT
CCTGATCTACCATACATCAAGATTACACTCAGGAGTCCCATCAAGGTTCAGTGGCAGTGGGTCTGGAACAGATTATTCTCTCA
CCATTAGCAACCTGGAGCAAGAAGATATTGCCACTTACTTTTGCCAACAGGGTAATACGCTTCCGTACACGTTCGGAGGGGGG
ACCAAGCTGGAGATCACAGGTGGCGGTGGCTCGGGCGGTGGTGGGTCGGGTGGCGGCGGATCTGAGGTGAAACTGCAGGAGTC
AGGACCTGGCCTGGTGGCGCCCTCACAGAGCCTGTCCGTCACATGCACTGTCTCAGGGGTCTCATTACCCGACTATGGTGTAA
GCTGGATTCGCCAGCCTCCACGAAAGGGTCTGGAGTGGCTGGGAGTAATATGGGGTAGTGAAACCACATACTATAATTCAGCT
CTCAAATCCAGACTGACCATCATCAAGGACAACTCCAAGAGCCAAGTTTTCTTAAAAATGAACAGTCTGCAAACTGATGACAC
AGCCATTTACTACTGTGCCAAACATTATTACTACGGTGGTAGCTATGCTATGGACTACTGGGGCCAAGGAACCTCAGTCACCG
TCTCCTCAACCACGACGCCAGCGCCGCGACCACCAACACCGGCGCCCACCATCGCGTCGCAGCCCCTGTCCCTGCGCCCAGAG
GCGTGCCGGCCAGCGGCGGGGGGCGCAGTGCACACGAGGGGGCTGGACTTCGCCTGTGATATCTACATCTGGGCGCCCTTGGC
CGGGACTTGTGGGGTCCTTCTCCTGTCACTGGTTATCACCCTTTACTGCAAACGGGGCAGAAAGAAACTCCTGTATATATTCA
AACAACCATTTATGAGACCAGTACAAACTACTCAAGAGGAAGATGGCTGTAGCTGCCGATTTCCAGAAGAAGAAGAAGGAGGA
TGTGAACTGAGAGTGAAGTTCAGCAGGAGCGCAGACGCCCCCGCGTACCAGCAGGGCCAGAACCAGCTCTATAACGAGCTCAA
TCTAGGACGAAGAGAGGAGTACGATGTTTTGGACAAGAGACGTGGCCGGGACCCTGAGATGGGGGGAAAGCCGAGAAGGAAGA
ACCCTCAGGAAGGCCTGTACAATGAACTGCAGAAAGATAAGATGGCGGAGGCCTACAGTGAGATTGGGATGAAAGGCGAGCGC
CGGAGGGGCAAGGGGCACGATGGCCTTTACCAGGGTCTCAGTACAGCCACCAAGGACACCTACGACGCCCTTCACATGCAGGC
CCTGCCCCCTCGCTGAGGATCCACGCGTTAAGTCGACAATCAACCTCTGGATTACAAAATTTGTGAAAGATTGACTGGTATTC
TTAACTATGTTGCTCCTTTTACGCTATGTGGATACGCTGCTTTAATGCCTTTGTATCATGCTATTGCTTCCCGTATGGCTTTC
ATTTTCTCCTCCTTGTATAAATCCTGGTTGCTGTCTCTTTATGAGGAGTTGTGGCCCGTTGTCAGGCAACGTGGCGTGGTGTG
CACTGTGTTTGCTGACGCAACCCCCACTGGTTGGGGCATTGCCACCACCTGTCAGCTCCTTTCCGGGACTTTCGCTTTCCCCC
TCCCTATTGCCACGGCGGAACTCATCGCCGCCTGCCTTGCCCGCTGCTGGACAGGGGCTCGGCTGTTGGGCACTGACAATTCC
GTGGTGTTGTCGGGGAAATCATCGTCCTTTCCTTGGCTGCTCGCCTGTGTTGCCACCTGGATTCTGCGCGGGACGTCCTTCTG
CTACGTCCCTTCGGCCCTCAATCCAGCGGACCTTCCTTCCCGCGGCCTGCTGCCGGCTCTGCGGCCTCTTCCGCGTCTTCGCC
TTCGCCCTCAGACGAGTCGGATCTCCCTTTGGGCCGCCTCCCCGCGTCGACTTTAAGACCAATGACTTACAAGGCAGCTGTAG
ATCTTAGCCACTTTTTAAAAGAAAAGGGGGGACTGGAAGGGCTAATTCACTCCCAACGAAGACAAGATCTGCTTTTTGCTTGT
ACTGGGTCTCTCTGGTTAGACCAGATCTGAGCCTGGGAGCTCTCTGGCTAACTAGGGAACCCACTGCTTAAGCCTCAATAAAG
CTTGCCTTGAGTGCTTCAAGTAGTGTGTGCCCGTCTGTTGTGTGACTCTGGTAACTAGAGATCCCTCAGACCCTTTTAGTCAG
TGTGGAAAATCTCTAGCA

SEQ ID NO:3     5'HIV R-U5-Δgag
GGGTCTCTCTGGTTAGACCAGATCTGAGCCTGGGAGCTCTCTGGCTAACTAGGGAACCCACTGCTTAAGCCTCAATAAAGCTT
GCCTTGAGTGCTTCAAGTAGTGTGTGCCCGTCTGTTGTGTGACTCTGGTAACTAGAGATCCCTCAGACCCTTTTAGTCAGTGT
GGAAAATCTCTAGCAGTGGCGCCCGAACAGGGACTTGAAAGCGAAAGGGAAACCAGAGGAGCTCTCTCGACGCAGGACTCGGC
TTGCTGAAGCGCGCACGGCAAGAGGCGAGGGGCGGCGACTGGTGAGTACGCCAAAAATTTTGACTAGCGGAGGCTAGAAGGAG
AGAGATGGGTGCGAGAGCGTCAGTATTAAGCGGGGGAGAATTAGATCGCGATGGGAAAAAATTCGGTTAAGGCCAGGGGGAAA
GAAAAAATATAAATTAAAACATATAGTATGGGCAAGCAGGGAGCTAGAACGATTCGCAGTTAATCCTGGCCTGTTAGAAACAT
CAGAAGGCTGTAGACAAATACTGGGACAGCTACAACCATCCCTTCAGACAGGATCAGAAGAACTTAGATCATTATATAATACA
GTAGCAACCCTCTATTGTGTGCATCAAAGGATAGAGATAAAAGACACCAAGGAAGCTTTAGACAAGATAGAGGAAGAGCAAAA
CAAAAGTAAGA

SEQ ID NO:4 HIV-psi (ψ)
GATCTTCAGACCTGGAGGAGGAGATATGAGGGACAATTGGAGAAGTGAATTATATAAATATAAAGTAGTAAAAATTGAACCAT
TAGGAGTAGCACCCACCAAGGCAAAGAGAAGAGTGGTGCAGAGAGAAAAAAGAGCAGTGGGAATAGGAGCTTTGTTCCTTGGG

TTCTTGGGAGCAGCAGGAAGCACTATGGGCGCAGCGTCAATGACGCTGACGGTACAGGCCAGACAATTATTGTCTGGTATAGT
GCAGCAGCAGAACAATTTGCTGAGGGCTATTGAGGCGCAACAGCATCTGTTGCAACTCACAGTCTGGGGCATCAAGCAGCTCC
AGGCAAGAATCCTGGCTGTGGAAAGATACCTAAAGGATCAACAGCTCCTGGGGATTTGGGGTTGCTCTGGAAAACTCATTTGC
ACCACTGCTGTGCCTTGGAATGCTAGTTGGAGTAATAAATCTCTGGAACAGATTTGGAATCACACGACCTGGATGGAGTGGGA
CAGAGAAATTAACAATTACACAAGCTTAATACACTCCTTAATTGAAGAATCGCAAAACCAGCAAGAAAAGAATGAACAAGAAT
TATTGGAATTAGATAAATGGGCAAGTTTGTGGAATTGGTTTAACATAACAAATTGGCTGTGGTATATAAAATTATTCATAATG
ATAGTAGGAGGCTTGGTAGGTTTAAGAATAGTTTTTGCTGTACTTTCTATAGTGAATAGAGTTAGGCAGGGATATTCACCATT
ATCGTTTCAGACCCACCTCCCAACCCCGAGGGGACCCGACAGGCCCGAAGGAATAGAAGAAGAAGGTGGAGAGAGAGACAGAG
ACAGATCCATTCGATTAGTGAACGGATC

SEQ ID NO:5 RRE
AGGAGCTTTGTTCCTTGGGTTCTTGGGAGCAGCAGGAAGCACTATGGGCGCAGCGTCAATGACGCTGACGGTACAGGCCAGAC
AATTATTGTCTGGTATAGTGCAGCAGCAGAACAATTTGCTGAGGGCTATTGAGGCGCAACAGCATCTGTTGCAACTCACAGTC
TGGGGCATCAAGCAGCTCCAGGCAAGAATCCTGGCTGTGGAAAGATACCTAAAGGATCAACAGCTCCT

SEQ ID NO:6 cPPT/CTS
TTTTAAAAGAAAAGGGGGGATTGGGGGGTACAGTGCAGGGGAAAGAATAGTAGACATAATAGCAACAGACATACAAACTAAAG
AATTACAAAAACAAATTACAAAAATTCAAAATTTT

SEQ ID NO:7 EF1α promoter long
TCCGGTGCCCGTCAGTGGGCAGAGCGCACATCGCCCACAGTCCCCGAGAAGTTGGGGGGAGGGGTCGGCAATTGAACCGGTGC
CTAGAGAAGGTGGCGCGGGGTAAACTGGGAAAGTGATGTCGTGTACTGGCTCCGCCTTTTTCCCGAGGGTGGGGGAGAACCGT
ATATAAGTGCAGTAGTCGCCGTGAACGTTCTTTTTCGCAACGGGTTTGCCGCCAGAACACAGCTGAAGCTTCGAGGGGCTCGC
ATCTCTCCTTCACGCGCCCGCCGCCCTACCTGAGGCCGCCATCCACGCCGGTTGAGTCGCGTTCTGCCGCCTCCCGCCTGTGG
TGCCTCCTGAACTGCGTCCGCCGTCTAGGTAAGTTTAAAGCTCAGGTCGAGACCGGGCCTTTGTCCGGCGCTCCCTTGGAGCC
TACCTAGACTCAGCCGGCTCTCCACGCTTTGCCTGACCCTGCTTGCTCAACTCTACGTCTTTGTTTCGTTTTCTGTTCTGCGC
CGTTACAGATCCAAGCTGTGACCGGCGCCTACG

SEQ ID NO:8 CAR-19 coding sequence
ATGGCCTTACCAGTGACCGCCTTGCTCCTGCCGCTGGCCTTGCTGCTCCACGCCGCCAGGCCGGACATCCAGATGACACAGAC
TACATCCTCCCTGTCTGCCTCTCTGGGAGACAGAGTCACCATCAGTTGCAGGGCAAGTCAGGACATTAGTAAATATTTAAATT
GGTATCAGCAGAAACCAGATGGAACTGTTAAACTCCTGATCTACCATACATCAAGATTACACTCAGGAGTCCCATCAAGGTTC
AGTGGCAGTGGGTCTGGAACAGATTATTCTCTCACCATTAGCAACCTGGAGCAAGAAGATATTGCCACTTACTTTTGCCAACA
GGGTAATACGCTTCCGTACACGTTCGGAGGGGGGACCAAGCTGGAGATCACAGGTGGCGGTGGCTCGGGCGGTGGTGGGTCGG
GTGGCGGCGGATCTGAGGTGAAACTGCAGGAGTCAGGACCTGGCCTGGTGGCGCCCTCACAGAGCCTGTCCGTCACATGCACT
GTCTCAGGGGTCTCATTACCCGACTATGGTGTAAGCTGGATTCGCCAGCCTCCACGAAAGGGTCTGGAGTGGCTGGGAGTAAT
ATGGGGTAGTGAAACCACATACTATAATTCAGCTCTCAAATCCAGACTGACCATCATCAAGGACAACTCCAAGAGCCAAGTTT
TCTTAAAAATGAACAGTCTGCAAACTGATGACACAGCCATTTACTACTGTGCCAAACATTATTACTACGGTGGTAGCTATGCT
ATGGACTACTGGGGCCAAGGAACCTCAGTCACCGTCTCCTCAACCACGACGCCAGCGCCGCGACCACCAACACCGGCGCCCAC
CATCGCGTCGCAGCCCCTGTCCCTGCGCCCAGAGGCGTGCCGGCCAGCGGCGGGGGGCGCAGTGCACACGAGGGGGCTGGACT
TCGCCTGTGATATCTACATCTGGGCGCCCTTGGCCGGGACTTGTGGGGTCCTTCTCCTGTCACTGGTTATCACCCTTTACTGC
AAACGGGGCAGAAAGAAACTCCTGTATATATTCAAACAACCATTTATGAGACCAGTACAAACTACTCAAGAGGAAGATGGCTG
TAGCTGCCGATTTCCAGAAGAAGAAGAAGGAGGATGTGAACTGAGAGTGAAGTTCAGCAGGAGCGCAGACGCCCCCGCGTACA
AGCAGGGCCAGAACCAGCTCTATAACGAGCTCAATCTAGGACGAAGAGAGGAGTACGATGTTTTGGACAAGAGACGTGGCCGG
GACCCTGAGATGGGGGGAAAGCCGAGAAGGAAGAACCCTCAGGAAGGCCTGTACAATGAACTGCAGAAAGATAAGATGGCGGA
GGCCTACAGTGAGATTGGGATGAAAGGCGAGCGCCGGAGGGGCAAGGGGCACGATGGCCTTTACCAGGGTCTCAGTACAGCCA
CCAAGGACACCTACGACGCCCTTCACATGCAGGCCCTGCCCCCTCGC

SEQ ID NO:9 WPRE
TAATCAACCTCTGGATTACAAAATTTGTGAAAGATTGACTGGTATTCTTAACTATGTTGCTCCTTTTACGCTATGTGGATACG
CTGCTTTAATGCCTTTGTATCATGCTATTGCTTCCCGTATGGCTTTCATTTTCTCCTCCTTGTATAAATCCTGGTTGCTGTCT
CTTTATGAGGAGTTGTGGCCCGTTGTCAGGCAACGTGGCGTGGTGTGCACTGTGTTTGCTGACGCAACCCCCACTGGTTGGGG
CATTGCCACCACCTGTCAGCTCCTTTCCGGGACTTTCGCTTTCCCCCTCCCTATTGCCACGGCGGAACTCATCGCCGCCTGCC
TTGCCCGCTGCTGGACAGGGGCTCGGCTGTTGGGCACTGACAATTCCGTGGTGTTGTCGGGGAAATCATCGTCCTTTCCTTGG
CTGCTCGCCTGTGTTGCCACCTGGATTCTGCGCGGGACGTCCTTCTGCTACGTCCCTTCGGCCCTCAATCCAGCGGACCTTCC
TTCCCGCGGCCTGCTGCCGGCTCTGCGGCCTCTTCCGCGTCTTCGCCTTCGCCCTCAGACGAGTCGGATCTCCCTTTGGGCCG
CCTCCCCGC

SEQ ID NO:10 3' SIN LTR
ATCGAGACCTAGAAAAACATGGAGCAATCACAAGTAGCAATACAGCAGCTACCAATGCTGATTGTGCCTGGCTAGAAGCACAA
GAGGAGGAGGAGGTGGGTTTTCCAGTCACACCTCAGGTACCTTTAAGACCAATGACTTACAAGGCAGCTGTAGATCTTAGCCA
CTTTTTAAAAGAAAAGGGGGGACTGGAAGGGCTAATTCACTCCCAACGAAGACAAGATATCCTTGATCTGTGGATCTACCACA
CACAAGGCTACTTCCCTGATTGGCAGAACTACACACCAGGGCCAGGGATCAGATATCCACTGACCTTTGGATGGTGCTACAAG
CTAGTACCAGTTGAGCAAGAGAAGGTAGAAGAAGCCAATGAAGGAGAGAACACCCGCTTGTTACACCCTGTGAGCCTGCATGG
GATGGATGACCCGGAGAGAGAAGTATTAGAGTGGAGGTTTGACAGCCGCCTAGCATTTCATCACATGGCCCGAGAGCTGCATC
CGGACTGTACTGGGTCTCTCTGGTTAGACCAGATCTGAGCCTGGGAGCTCTCTGGCTAACTAGGGAACCCACTGCTTAAGCCT

CAATAAAGCTTGCCTTGAGTGCTTCAAGTAGTGTGTGCCCGTCTGTTGTGTGACTCTGGTAACTAGAGATCCCTCAGACCCTT
TTAGTCAGTGTGGAAAATCTCTAGCA

SEQ ID NO:11 truncated 5'LTR
GGGTCTCTCTGGTTAGACCAGATCTGAGCCTGGGAGCTCTCTGGCTAACTAGGGAACCCACTGCTTAAGCCTCAATAAAGCTT
GCCTTGAGTGCTTCAAGTAGTGTGTGCCCGTCTGTTGTGTGACTCTGGTAACTAGAGATCCCTCAGACCCTTTTAGTCAGTGT
GGAAAATCTCTAGCA

SEQ ID NO:12 HIV-psi (ψ)
CTCTCTCGACGCAGGACTCGGCTTGCTGAAGCGCGCACGGCAAGAGGCGAGGGGCGGCGACTGGTGAGTACGCCAAAAATTTT
GACTAGCGGAGGCTAGAAGGAGAGAGATGGGTGCGAGAGCGTC

SEQ ID NO:13 EF1α promoter short
GGGCAGAGCGCACATCGCCCACAGTCCCCGAGAAGTTGGGGGGAGGGGTCGGCAATTGATCCGGTGCCTAGAGAAGGTGGCGC
GGGGTAAACTGGGAAAGTGATGTCGTGTACTGGCTCCGCCTTTTTCCCGAGGGTGGGGGAGAACCGTATATAAGTGCAGTAGT
CGCCGTGAACGTTCTTTTTCGCAACGGGTTTGCCGCCAGAACACAG

SEQ ID NO:14 WPRE
AATCAACCTCTGGATTACAAAATTTGTGAAAGATTGACTGGTATTCTTAACTATGTTGCTCCTTTTACGCTATGTGGATACGC
TGCTTTAATGCCTTTGTATCATGCTATTGCTTCCCGTATGGCTTTCATTTTCTCCTCCTTGTATAAATCCTGGTTGCTGTCTC
TTTATGAGGAGTTGTGGCCCGTTGTCAGGCAACGTGGCGTGGTGTGCACTGTGTTTGCTGACGCAACCCCCACTGGTTGGGGC
ATTGCCACCACCTGTCAGCTCCTTTCCGGGACTTTCGCTTTCCCCCTCCCTATTGCCACGGCGGAACTCATCGCCGCCTGCCT
TGCCCGCTGCTGGACAGGGGCTCGGCTGTTGGGCACTGACAATTCCGTGGTGTTGTCGGGGAAATCATCGTCCTTTCCTTGGC
TGCTCGCCTGTGTTGCCACCTGGATTCTGCGCGGGACGTCCTTCTGCTACGTCCCTTCGGCCCTCAATCCAGCGGACCTTCCT
TCCCGCGGCCTGCTGCCGGCTCTGCGGCCTCTTCCGCGTCTTCGCCTTCGCCCTCAGACGAGTCGGATCTCCCTTTGGGCCGC
CTCCCCGC

SEQ ID NO:15 3' LTR Δ-U3
TGGAAGGGCTAATTCACTCCCAACGAAGACAAGATCTGCTTTTTGCTTGTACTGGGTCTCTCTGGTTAGACCAGATCTGAGCC
TGGGAGCTCTCTGGCTAACTAGGGAACCCACTGCTTAAGCCTCAATAAAGCTTGCCTTGAGTGCTTCAAGTAGTGTGTGCCCG
TCTGTTGTGTGACTCTGGTAACTAGAGATCCCTCAGACCCTTTTAGTCAGTGTGGAAAATCTCTAGCA

SEQ ID NO:16     CAR-19vamino acid sequence
MALPVTALLL PLALLLHAAR PDIQMTQTTS SLSASLGDRV TISCRASQDI SKYLNWYQQK PDGTVKLLIY HTSRLHSGVP
SRFSGSGSGT DYSLTISNLE QEDIATYFCQ QGNTLPYTFG GGTKLEITGG GGSGGGGSGG GGSEVKLQES GPGLVAPSQS
LSVTCTVSGV SLPDYGVSWI RQPPRKGLEW LGVIWGSETT YYNSALKSRL TIIKDNSKSQ VFLKMNSLQT DDTAIYYCAK
HYYYGGSYAM DYWGQGTSVT VSSTTTPAPR PPTPAPTIAS QPLSLRPEAC RPAAGGAVHT RGLDFACDIY IWAPLAGTCG
VLLLSLVITL YCKRGRKKLL YIFKQPFMRP VQTTQEEDGC SCRFPEEEEG GCELRVKFSR SADAPAYQQG QNQLYNELNL
GRREEYDVLD KRRGRDPEMG GKPRRKNPQE GLYNELQKDK MAEAYSEIGM KGERRRGKGH DGLYQGLSTA TKDTYDALHM
QALPPR

SEQ ID NO:17 amino acid sequence of DNRII
MGRGLLRGLW PLHIVLWTRI ASTIPPHVQK SVNNDMIVTD NNGAVKFPQL CKFCDVRFST CDNQKSCMSN CSITSICEKP
QEVCVAVWRK NDENITLETV CHDPKLPYHD FILEDAASPK CIMKEKKKPG ETFFMCSCSS DECNDNIIFS EEYNTSNPDL
LLVIFQVTGI SLLPPLGVAI SVIIIFYCYR VNRQQKLSST WETGKTRKLM EFSEHCAII

SEQ ID NO:18 nucleotide sequence of DNRII
ATGGGTCGGG GGCTGCTCAG GGGCCTGTGG CCGCTGCACA TCGTCCTGTG GACGCGTATC GCCAGCACGA TCCCACCGCA
CGTTCAGAAG TCGGTTAATA ACGACATGAT AGTCACTGAC AACAACGGTG CAGTCAAGTT TCCACAACTG TGTAAATTTT
GTGATGTGAG ATTTTCCACC TGTGACAACC AGAAATCCTG CATGAGCAAC TGCAGCATCA CCTCCATCTG TGAGAAGCCA
CAGGAAGTCT GTGTGGCTGT ATGGAGAAAG AATGACGAGA ACATAACACT AGAGACAGTT TGCCATGACC CCAAGCTCCC
CTACCATGAC TTTATTCTGG AAGATGCTGC TTCTCCAAAG TGCATTATGA AGGAAAAAAA AAAGCCTGGT GAGACTTTCT
TCATGTGTTC CTGTAGCTCT GATGAGTGCA ATGACAACAT CATCTTCTCA GAAGAATATA ACACCAGCAA TCCTGACTTG
TTGCTAGTCA TATTTCAAGT GACAGGCATC AGCCTCCTGC CACCACTGGG AGTTGCCATA TCTGTCATCA TCATCTTCTA
CTGCTACCGC GTTAACCGGC AGCAGAAGCT GAGTTCAACC TGGGAAACCG GCAAGACGCG GAAGCTCATG GAGTTCAGCG
AGCACTGTGC CATCATC

## Claims

1. A lentiviral vector comprising a truncated EF1α promoter for directing the expression of a nucleotide sequence encoding a polypeptide of interest in a host cell, for example, the truncated EF1α promoter is an EF1α core promoter comprising the nucleotide sequence setting forth in SEQ ID NO: 13.

2. The lentiviral vector of claim 1, wherein the lentiviral vector is a non-replicating lentiviral vector.

3. The lentiviral vector of claim 1 or 2, further comprises an element selecting from the group consisting of a 5'LTR, a ψ element, an RRE element, a cPPT/CTS element, a truncated EF1α promoter, a WPRE element, a multiple cloning site for inserting the nucleotide sequence encoding the polypeptide of interest, and a 3'LTR.

4. The lentiviral vector of any one of claim 1-3, which comprises a 5'LTR, a ψ element, an RRE element, a cPPT/CTS element, a truncated EF1α promoter, a WPRE element, a 3'LTR, and optionally, a multiple cloning site for inserting the nucleotide sequence encoding the polypeptide of interest, which are operably linked.

5. The lentiviral vector of claim 3 or 4, wherein the 5'LTR comprises the nucleotide sequence set forth in SEQ ID NO: 3 or 11; the ψ element comprises the nucleotide sequence set forth in SEQ ID NO: 4 or 12; the RRE element comprises the nucleotide sequence set forth in SEQ ID NO: 5; the cPPT/CTS element comprises the nucleotide sequence set forth in SEQ ID NO: 6; the WPRE element comprises the nucleotide sequence set forth in SEQ ID NO: 9 or 14; the 3'LTR comprises the nucleotide sequence set forth in SEQ ID NO: 10 or 15.

6. The lentiviral vector of any one of claims 1-5, which comprises a 5'LTR comprising the nucleotide sequence set forth in SEQ ID NO: 11, a ψ element comprising the nucleotide sequence set forth in SEQ ID NO: 12, an RRE element comprising the nucleotide sequence set forth in SEQ ID NO: 5, a cPPT/CTS element comprising the nucleotide sequence set forth in SEQ ID NO: 6, a truncated EF1α promoter comprising the nucleotide sequence set forth in SEQ ID NO: 13, a WPRE element comprising the nucleotide sequence set forth in SEQ ID NO: 14, a 3'LTR comprising the nucleotide sequence set forth in SEQ ID NO: 15, and optionally, a multiple cloning site for inserting the nucleotide sequence encoding the polypeptide of interest, which are operably linked.

7. The lentiviral vector of any one of claims 1 to 6, further comprising a nucleotide sequence encoding a polypeptide of interest.

8. The lentiviral vector of any one of claims 1 to 7, wherein the polypeptide of interest is a fusion polypeptide comprising a plurality of proteins, and the plurality of proteins in the fusion polypeptide are separated by a self-cleavable peptide.

9. The lentiviral vector of any one of claims 1 to 8, wherein the polypeptide of interest is a fusion polypeptide comprising a first protein and a second protein, and the fusion polypeptide comprises a self-cleavable peptide between the first protein and the second protein.

10. The lentiviral vector of claim 8 or 9, wherein the self-cleavable peptide is a 2A polypeptide, for example, the self-cleavable peptide is selected from P2A, F2A, E2A, or T2A polypeptide, or a functional variant thereof.

11. The lentiviral vector of claim 9 or 10, wherein the first protein is a cancer-associated antigen-specific receptor protein, such as T cell receptor (TCR) or chimeric antigen receptor (CAR).

12. The lentiviral vector of any one of claims 9-11, wherein the second protein is a dominant negative TGF-β type II receptor.

13. The lentiviral vector of claim 12, wherein the dominant negative TGF-β type II receptor lacks the intracellular signaling domain of TGF-β type II receptor, for example, the dominant negative TGF-β type II receptor comprises the amino acid sequence set forth in SEQ ID NO:18.

14. A method for preparing a lentiviral vector particle, comprising:
a) co-transfecting a suitable host cell with the lentiviral vector of any one of claims 1-10, one or more packaging vectors expressing Gag and/or Pol, and an envelope vector expressing an envelope protein such as VSV-G;
b) culturing the transfected host cell to package the lentiviral vector into a lentiviral vector particle; and
c) harvesting the lentiviral vector particle produced in step b).

15. A lentiviral vector particle comprising the lentiviral vector of any one of claims 1-13 or prepared by the method of claim 14.

16. Use of the lentiviral vector particle of claim 15 in the preparation of a therapeutic T cell, wherein the therapeutic T cell expresses a cancer-associated antigen-specific receptor protein, such as T cell receptor (TCR) or chimeric antigen receptor (CAR), and optionally a dominant negative TGF-β type II receptor.

17. A method for preparing a therapeutic T cell, comprising transducing a T cell with the lentiviral vector particle of claim 15.

18. The method of claim 17, wherein the transduction of the lentiviral vector particle causes the therapeutic T cell to express a cancer-associated antigen-specific receptor protein, such as T cell receptor (TCR) or chimeric antigen receptor (CAR), and optionally a dominant negative TGF-β type II receptor.

## Amended claims

### Amended claims under Art. 19.1 PCT

1. A lentiviral vector comprising a truncated EF1α promoter for directing the expression of a nucleotide sequence encoding a polypeptide of interest in a host cell, for example, the truncated EF1α promoter is an EF1α core promoter comprising the nucleotide sequence setting forth in SEQ ID NO: 13,
wherein the lentiviral vector is a non-replicating lentiviral vector, and
wherein the lentiviral vector comprises a 5'LTR, a ψ element, an RRE element, a cPPT/CTS element, a truncated EF1α promoter, a WPRE element, a 3'LTR, and optionally, a multiple cloning site for inserting the nucleotide sequence encoding the polypeptide of interest, which are operably linked.

2. The lentiviral vector of claim 1, wherein the 5'LTR comprises the nucleotide sequence set forth in SEQ ID NO: 3 or 11; the ψ element comprises the nucleotide sequence set forth in SEQ ID NO: 4 or 12; the RRE element comprises the nucleotide sequence set forth in SEQ ID NO: 5; the cPPT/CTS element comprises the nucleotide sequence set forth in SEQ ID NO: 6; the WPRE element comprises the nucleotide sequence set forth in SEQ ID NO: 9 or 14; the 3'LTR comprises the nucleotide sequence set forth in SEQ ID NO: 10 or 15.

3. The lentiviral vector of any one of claims 1-2, which comprises a 5'LTR comprising the nucleotide sequence set forth in SEQ ID NO: 11, a ψ element comprising the nucleotide sequence set forth in SEQ ID NO: 12, an RRE element comprising the nucleotide sequence set forth in SEQ ID NO: 5, a cPPT/CTS element comprising the nucleotide sequence set forth in SEQ ID NO: 6, a truncated EF1α promoter comprising the nucleotide sequence set forth in SEQ ID NO: 13, a WPRE element comprising the nucleotide sequence set forth in SEQ ID NO: 14, a 3'LTR comprising the nucleotide sequence set forth in SEQ ID NO: 15, and optionally, a multiple cloning site for inserting the nucleotide sequence encoding the polypeptide of interest, which are operably linked.

4. The lentiviral vector of any one of claims 1 to 3, further comprising a nucleotide sequence encoding a polypeptide of interest.

5. The lentiviral vector of any one of claims 1 to 4, wherein the polypeptide of interest is a fusion polypeptide comprising a plurality of proteins, and the plurality of proteins in the fusion polypeptide are separated by a self-cleavable peptide.

6. The lentiviral vector of any one of claims 1 to 5, wherein the polypeptide of interest is a fusion polypeptide comprising a first protein and a second protein, and the fusion polypeptide comprises a self-cleavable peptide between the first protein and the second protein.

7. The lentiviral vector of claim 5 or 6, wherein the self-cleavable peptide is a 2A polypeptide, for example, the self-cleavable peptide is selected from P2A, F2A, E2A, or T2A polypeptide, or a functional variant thereof.

8. The lentiviral vector of claim 6 or 7, wherein the first protein is a cancer-associated antigen-specific receptor protein, such as T cell receptor (TCR) or chimeric antigen receptor (CAR).

9. The lentiviral vector of any one of claims 6-8, wherein the second protein is a dominant negative TGF-β type II receptor.

10. The lentiviral vector of claim 9, wherein the dominant negative TGF-β type II receptor lacks the intracellular signaling domain of TGF-β type II receptor, for example, the dominant negative TGF-β type II receptor comprises the amino acid sequence set forth in SEQ ID NO:18.

11. A method for preparing a lentiviral vector particle, comprising:
a) co-transfecting a suitable host cell with the lentiviral vector of any one of claims 1-7, one or more packaging vectors expressing Gag and/or Pol, and an envelope vector expressing an envelope protein such as VSV-G;
b) culturing the transfected host cell to package the lentiviral vector into a lentiviral vector particle; and
c) harvesting the lentiviral vector particle produced in step b).

12. A lentiviral vector particle comprising the lentiviral vector of any one of claims 1-10 or prepared by the method of claim 11.

13. Use of the lentiviral vector particle of claim 12 in the preparation of a therapeutic T cell, wherein the therapeutic T cell expresses a cancer-associated antigen-specific receptor
protein, such as T cell receptor (TCR) or chimeric antigen receptor (CAR), and optionally a dominant negative TGF-β type II receptor.

14. A method for preparing a therapeutic T cell, comprising transducing a T cell with the lentiviral vector particle of claim 12.

15. The method of claim 14, wherein the transduction of the lentiviral vector particle causes the therapeutic T cell to express a cancer-associated antigen-specific receptor protein, such as T cell receptor (TCR) or chimeric antigen receptor (CAR), and optionally a dominant negative TGF-β type II receptor.
